# FASCICULE DE BREVET EUROPEEN

(11) **EP 2 459 548 B1**
(45) Date de publication et mention de la délivrance du brevet: **19.11.2014**
(21) Numéro de dépôt: 10752089.2
(22) Date de dépôt: 23.07.2010
(51) Int. Cl.: C07D 401/10, C07D 401/12, A61K 31/495, A61P 3/00

(54) **DÉRIVÉS D'URÉE DE TETRAHYDROQUINOXALINE, LEUR PRÉPARATION ET LEUR APPLICATION EN THÉRAPEUTIQUE**
TETRAHYDROCHINOXALINHARNSTOFFDERIVATE, IHRE HERSTELLUNG UND THERAPEUTISCHE VERWENDUNG
TETRAHYDROQUINOXALINE UREA DERIVATIVES, PREPARATION THEREOF, AND THERAPEUTIC USE THEREOF

(30) Priorité: 27.07.2009 FR 0903685
(43) Date de publication de la demande: 06.06.2012
(73) Titulaire: SANOFI, 75008 Paris (FR)
(72) Inventeur: CRESPIN, Olivier, F-75013 Paris (FR); NICOLAI, Eric, F-75013 Paris (FR); VENIER, Olivier, F-75013 Paris (FR)
(74) Mandataire: Böhm, Brigitte
(86) Numéro de dépôt international: PCT/FR2010/051563
(87) Numéro de publication internationale: WO 2011/012800

(56) Documents cités:
- WO-A2-2008/000950
- WO-A2-2008/000951
- WO-A2-2009/112691
- HUGHES, K.A., WEBSTER, S.P., WALKER, B.R.: "11-Beta-hydroxysteroid dehydrogenase type 1 (11beta-HSD1) inhibitors in Type 2 diabetes mellitus and obesity" EXPERT OPIN. INVESTIG. DRUGS, vol. 17, no. 4, 2008, pages 481-496, XP002563741
- SAIAH, E.: "The role of 11beta-hydroxysteroid dehydrogenase in metabolic Disease and Therapeutic Potential of 11beta-HSD1 Inhibitors" CURRENT MEDICINAL CHEMISTRY, vol. 15, 2008, pages 642-649, XP002508148

## Description

La présente invention se rapporte à des dérivés d'urée de tétrahydroquinoxaline, à leur préparation et à leur application en thérapeutique.

Les composés selon l'invention modulent l'activité de la 11β-hydroxystéroïde déshydrogénase type 1 (11βHSD1) et sont utiles pour le traitement des pathologies dans lesquelles une telle modulation est bénéfique, comme dans le cas du syndrome métabolique ou du diabète de type 2 non insulino dépendant.

La 11βHSD1 catalyse localement la conversion de glucocorticoïdes inactifs (cortisone chez l'Homme) en glucocorticoïdes actifs (cortisol chez l'Homme) dans différents tissus et organes, principalement le foie et le tissu adipeux, mais aussi dans les muscles, les os, le pancréas, l'endothélium, le tissu oculaire et dans certaines parties du système nerveux central. La 11βHSD1 agit comme un régulateur de l'action des glucocorticoïdes dans les tissus et organes où elle est exprimée (Tomlinson et al., Endocrine Reviews 25(5), 831-866 (2004), Davani et al., J. Biol. Chem. 275, 34841 (2000) ; Moisan et al., Endocrinology, 127, 1450 (1990)).

Les principales pathologies dans lesquelles interviennent les glucocorticoïdes et l'inhibition de la 11βHSD1 sont indiquées ci-après.

### A. Obésité, diabète de type 2 et syndrome métabolique

Le rôle de la 11βHSD1 dans l'obésité, le diabète de type 2 et le syndrome métabolique (aussi connu sous le nom de syndrome X ou syndrome de résistance à l'insuline) où les symptômes incluent l'obésité viscérale, l'intolérance au glucose, la résistance à l'insuline, l'hypertension, le diabète de type 2 et l'hyperlipidémie (Reaven Ann. Rev. Med 44, 121 (1993)) est décrit dans de nombreuses publications. Chez l'Homme, le traitement par la carbenoxolone (un inhibiteur non spécifique de la 11βHSD1) améliore la sensibilité à l'insuline chez des patients volontaires minces et chez des diabétiques de type 2 (Andrews et al., J. Clin. Endocrinol. Metab. 88, 285 (2003)). De plus, les souris dont le gène de la 11βHSD1 a été éteint sont résistantes à l'hyperglycémie induite par le stress et l'obésité, montrent une atténuation de l'induction d'enzymes hépatiques de la néoglucogenèse (PEPCK et G6P) et présentent une augmentation de la sensibilité à l'insuline dans le tissu adipeux (Kotelevstev et al., Proc. Nat Acad. Sci. 94, 14924 (1997) ; Morton et al., J. Biol. Chem. 276, 41293 (2001)). Par ailleurs, les souris transgéniques où le gène de la 11βHSD1 a été surexprimé dans les tissus adipeux présentent un phénotype similaire à celui du syndrome métabolique humain (Masuzaki et al., Science 294, 2166 (2001)). Il est à noter que le phénotype observé existe sans une augmentation du total des glucocorticoïdes circulants, mais est induit par l'augmentation spécifique de glucocorticoïdes actifs dans les dépôts adipeux.

Par ailleurs, de nouvelles classes d'inhibiteurs spécifiques de la 11βHSD1 sont apparues récemment :
- des arylsulfonamidothiazoles ont montré qu'ils amélioraient la sensibilité à l'insuline et réduisaient le niveau de glucose dans le sang de souris présentant une hyperglycémie (Barf et al., J. Med. Chem. 45, 3813 (2002)). De plus, dans une étude récente, il a été montré que ce type de composés réduisait la prise de nourriture ainsi que la prise de poids chez des souris obèses (Wang et Coll. Diabetologia 49, 1333 (2006*)*) ;
- des triazoles ont montré qu'ils amélioraient le syndrome métabolique et ralentissaient la progression de l'athérosclérose chez des souris (Hermanowski-Vosatka et al., J. Exp. Med. 202, 517 (2005)).

### A2. Complications microvasculaires du diabète

La présence des complications chroniques chez le diabétique de type 2 est souvent associée à la sévérité et à la durée du diabète. Les troubles microvasculaires fonctionnels et structuraux expliquent en grande partie le développement de certaines pathologies observées chez le diabétique telles la neuropathie, la rétinopathie, et la néphropathie (Rayman, Diabetes Review 7 :261-274, 1999 ; Gärtner and Eigentler, Clin Nephrol 70 :1-9, 2008 ; Zent and Pozzi, Sem Nephrol 27 :161-171, 2007; Malecki et al., EJCl38 :925-930, 2008). L'élévation chronique de la glycémie, ou l'intolérance au glucose, représentent des facteurs de risque majeurs de ces complications microvasculaires (Robinson Singleton et al. Diabetes 52 :2867-2873, 2003 ; Lachin et al. Diabetes 57: 995-1001, 2008). En permettant un meilleur contrôle de la glycémie, grâce à une baisse de la néoglucogénèse hépatique et une augmentation de la sensibilité à l'insuline de l'organisme (voir chapitre « obésité, diabète de type 2 et syndrome métabolique »), des inhibiteurs de 11β-HSD1 peuvent éviter la progression vers les complications microvasculaires observées chez le diabétique. Cependant, le strict contrôle de la glycémie ne permet pas d'éviter entièrement le développement des complications microvasculaires, et rend nécessaire la découverte de nouveaux traitements permettant traiter plus globalement les patients diabétiques et dyslipidémiques (Girach et al. Int J Clin Pract 60(11) : 1471-1483, 2006 ; Taylor. Curr Diab Rep 8 (5) : 345-352 ; 2008). De manière intéressante, une étude de Chiodini et al. (Diabetes Care 30 : 83-88, 2007) a montré que la sécrétion de cortisol, chez les patients diabétiques, était directement associée à la présence de complications macro- ou micro-vasculaires chroniques. Par ailleurs, la réactivité microvasculaire et la fonction endothéliale sont altérées chez le patient souffrant du syndrome de Cushing, présentant un hypercortisolisme (Prazny et al. Physiol Rev 57 : 13-22, 2008).

Plus particulièrement, Bhatia et al. (Ann Ophtalmol 15 :128-130 ; 1983) ont montré une association entre les taux de cortisol plasmatiques élevés et la rétinopathie chez le diabétique.

Koh et al. ont montré que le traitement de patients atteints du syndrome de Cushing par adrénalectomie, permettant de réverser l'hypercortisolisme, améliore la fonction rénale.

Les paramètres cliniques de polyneuropathies (perception sensorielle, neuropathie autonome cardiaque) sont associés à une augmentation de la sécrétion de cortisol chez les patients diabétiques (Tsigos et al. J Clin Endocrinol Metab 76 :554-558, 1993).

Tous ces éléments montrent qu'une diminution de l'impact du cortisol par une inhibition locale de sa régénération, via des inhibiteurs de 11β-HSD1, pourrait avoir un rôle favorable dans les troubles de la microcirculation associés au diabète (polyneuropathie, rétinopathie, et la néphropathie).

### B. Cognition et démence

Les troubles cognitifs légers sont des phénomènes communs aux personnes âgées et aux diabétiques de type 1 et 2, et peuvent conduire progressivement à la dépression ou la démence (Messier et al., Neurobiol. aging 26, 26; Greenwood et al.(2005), Neurobiol. aging 26, 45 (2005)). Autant dans le cas d'animaux que d'humains âgés, les différences inter-individuelles pour les fonctions cognitives générales ont été reliées aux différences d'exposition à long terme aux glucocorticoïdes (Lupien et al., Nat. Neurosci. 1, 69, (1998)). Par ailleurs, la dérégulation de l'axe HPA (hypothalamo-hypophyso-surrénalien)) résultant dans l'exposition chronique aux glucocorticoïdes de certaines sous-régions du cerveau a été proposée comme contribuant au déclin des fonctions cognitives (Mc Ewen et al., Curr. Opin. Neurobiol. 5, 205, 1995). La 11βHSD1 est abondante dans le cerveau et est exprimée dans de nombreuses sous régions incluant l'hypothalamus, le cortex frontal et le cerebellum (Sandeep et al., Proc. Natl. Acad. Sci. 101, 6734 (2004)). Les souris déficientes en 11βHSD1 sont protégées contre les dysfonctionnements de l'hypothalamus associés aux glucocorticoïdes qui sont rattachés à la vieillesse (Yau et al., Proc. Nati. Acad. Sci. 98, 4716, (2001)). De plus, dans des études chez l'Homme, il a été montré que l'administration de la carbenoxolone améliore la fluidité verbale et la mémoire verbale chez les personnes âgées (Yau et al., Proc. Natl. Acad. Sci. 98, 4716 (2001), Sandeep et al., Proc. Natl. Acad. Sci. 101, 6734 (2004)). Finalement, l'utilisation d'inhibiteurs sélectifs de la 11βHSD1 de type triazole a montré qu'ils prolongeaient la rétention de la mémoire chez des souris âgées (Rocha et al., Abstract 231 ACS meeting, Atlanta, 26-30 Mars 2006). Par ailleurs, il a été montré dans des modèles de rongeurs diabétiques que le taux de corticostérone contribuait au développement des pathologies cognitives induites par le diabète (Stranhan et al., Nature neurosc. 11,309 (2008)). Anisi, des inhibiteurs de la 11βHSD1, permettant une réduction de la régénération du cortisol au niveau de l'hippocampe, pourraient avoir un rôle bénéfique sur les fonctions cognitives chez les patients diabétiques âgés (Sandeep et al., Proc. Natl. Acad. Sci. 101, 6734 (2004)).

### C. Pression intra-oculaire

Les glucocorticoïdes peuvent être utilisés par voies topique ou systémique pour une grande variété de pathologies de l'ophtalmologie clinique. Une complication particulière de ces traitements est le glaucome induit par l'utilisation de corticostéroïdes. Cette pathologie est caractérisée par l'augmentation de la pression intra-oculaire (PIO). Dans les cas les plus graves et pour les formes non traitées, la PIO peut conduire à une perte de champ de vision partielle et éventuellement à une perte totale de la vision. La PIO est le résultat d'un déséquilibre entre la production d'humeur aqueuse et son drainage. L'humeur aqueuse est produite dans les cellules épithéliales non-pigmentées et le drainage est réalisé au travers des cellules du réseau trabéculaire. La 11βHSD1 est localisée dans les cellules épithéliales non-pigmentées et sa fonction est clairement l'amplification de l'activité des glucocorticoïdes dans ces cellules (Stokes et al., Invest. Ophthalmol. Vis. Sci. 41, 1629 (2000)). Cette notion est confirmée par l'observation que la concentration en cortisol libre est fortement excédentaire par rapport à la cortisone dans l'humeur aqueuse (ratio 14/1). L'activité fonctionnelle de la 11βHSD1 dans les yeux a été évaluée en étudiant l'action de la carbenoxolone chez des volontaires sains. Après sept jours de traitement à la carbenoxolone, la PIO est réduite de 18% (Rauz et al., Invest. Ophtamol. Vis. Sci. 42, 2037 (2001)). L'inhibition de la 11βHSD1 dans les yeux est donc prédite comme réduisant la concentration locale en glucocorticoïdes et la PIO, produisant un effet bénéfique dans le traitement du glaucome et d'autres désordres de la vision.

### D. Hypertension

Les substances hypertensives issues des adipocytes comme la leptine et l'angiotensinogène ont été proposées comme étant des éléments clés dans les pathologies d'hypertension reliées à l'obésité (Wajchenberg et al., Endocr. Rev. 21, 697 (2000)). La leptine qui est secrétée en excès chez les souris aP2-11βHSD1 transgéniques (Masuzaki et al., J. Clinical Invest. 112, 83 (2003)), peut activer différents réseaux de systèmes neuronaux sympathiques, incluant ceux qui régulent la pression artérielle (Matsuzawa et al., Acad. Sci. 892, 146 (1999)). De plus, le système rénine-angiotensine (SRA) a été identifié comme étant une voie déterminante dans la variation de la pression artérielle. L'angiotensinogène, qui est produit dans le foie et le tissu adipeux, est un substrat-clé pour la rénine et est à l'origine de l'activation du SRA. Le niveau plasmatique en angiotensiogène est significativement élevé dans les souris aP2-11βHSD1 transgéniques, comme le sont ceux de l'angiotensine II et de l'aldostérone (Masuzaki et al., J. Clinical Invest. 112, 83 (2003)) ; ces éléments conduisent à l'élévation de la pression artérielle. Le traitement de ces souris par de faibles doses d'un antagoniste du récepteur de l'angiotensine II abolit cette hypertension (Masuzaki et al., J. Clinical Invest. 112, 83 (2003)). Ces informations illustrent l'importance de l'activation locale des glucocorticoïdes dans le tissu adipeux et le foie, et suggère que cette hypertension puisse être causée ou exacerbée par l'activité de la 11βHSD1 dans ces tissus. L'inhibition de la 11βHSD1 et la réduction du niveau de glucocorticoïdes dans le tissu adipeux et/ou dans le foie est donc prédit comme ayant un rôle bénéfique pour le traitement de l'hypertension et des pathologies cardiovasculaires associées.

### D2. Hypertension artérielle sensible au sel

On estime qu'environ 30 à 50% de la population générale présente une sensibilité particulière au sel. De nombreuses évidences suggèrent un lien entre la sensibilité au sel et l'hypertension artérielle et les risques cardiovasculaires (Weinberger MH, Curr Opin Cardiol 2004 ; 19 :353-356). Il a été montré que les sujets sensibles au sel présentent une variabilité de la fréquence cardiaque diminuée, ainsi qu'une pression artérielle et une production de cortisol augmentée durant un stress mental, comparativement à des sujets non sensibles (Weber CS et al., Journal of Human Hypertension 2008 ; 22 :423-431). Par ailleurs, une récente étude de Liu Y et al. (Physiol Genomics 2008 Sept 30) a démontré chez le rat sensible au sel de Dahl (Dahl salt-sensitive rat), que l'inhibition spécifique de l'expression de la 11βHSD1 médullaire rénale, par l'utilisation de shRNA, permet de diminuer très nettement chez les animaux l'élévation de la pression artérielle moyenne induite par un régime salé. Ces éléments permettent de penser qu'un inhibiteur de l'enzyme 11βHSD1 aurait très probablement un effet bénéfique sur cette forme d'hypertension artérielle.

### E. Ostéoporose

Le développement du squelette et les fonctions osseuses sont aussi régulées par l'action des glucocorticoïdes. La 11βHSD1 est présente dans les ostéoclastes et ostéoblastes. Le traitement de volontaires sains par la carbenoxolone a montré une diminution des marqueurs de résorption osseuse sans changement dans les marqueurs de formation des os (Cooper et al., Bone, 27, 375 (2000)). L'inhibition de la 11βHSD1 et la réduction du niveau de glucocorticoïdes dans les os pourraient donc être utilisées comme un mécanisme de protection dans le traitement de l'ostéoporose.

### F. Lipodystrophie associée à une thérapie antirétrovirale hautement active (HAART), ou HAL syndrome.

L'utilisation d'un traitement antiretroviral intensif pour les patients atteints du sida induit souvent un syndrome de lipodystrophie (HAL) ressemblant au syndrome de Cushing, et associant augmentation de la masse grasse abdominale, hypertriglycéridémie et résistance à l'insuline. Il a été montré (Sutinen et al., Diabetologia, 47,1668 (2004)) que cette lipodystrophie (HAL) était associée à une augmentation de l'expression de la 11βHSD1 dans le tissu adipeux des patients. Des inhibiteurs de la 11βHSD1, permettant une réduction de la régénération du cortisol au niveau du tissu adipeux, pourraient donc avoir un rôle bénéfique chez les patients atteints de lipodystrophie associée à un traitement intensif du sida par des antitretroviraux (HAL syndrome).

### G. Maladies infectieuses

Certaines infections, comme la tuberculose, sont associées à des dérèglements de la réponse immunitaire (Ellner JJ, J. Lab. Clin. Med, 130, 469, (1997)). Cette particularité, qui s'accompagne le plus souvent de l'augmentation de la sécrétion de certaines cytokines (IL-10, TNFα) et/ou de la réponse à certaines cytokines, semble trouver, au moins en partie, sa cause dans l'exposition tissulaire locale des cellules immunitaires aux glucocorticoïdes. Par ailleurs, l'administration de glucocorticoïdes de synthèse chez l'homme ou l'animal provoque la réactivation de la tuberculose chez l'homme et chez l'animal (Haanas OC et al. Eur. J. Repir. Dis. 64, 294 (1998), Brown et al. Infect. Immun. 63, 2243, (1995)). De même, les stress divers, activateurs de l'axe HPA, ont pour conséquence une réactivation de cette infection.

En dehors de ces cas particuliers, les taux circulants de glucocorticoïdes ainsi que l'activation de l'axe HPA, semblent être normaux chez des patients atteints de tuberculose (Baker et al. Am. J Resp.Crit. Care. Med., 162, 1641 (2000)). Par contre, les taux de cortisol versus cortisone dans le liquide bronchoalvéolaire, semblent augmentés, traduisant une modulation du métabolisme des glucocorticoïdes vers la forme active (notamment dépendante de l'activité de la 11βHSD1). L'inhibition de la 11βHSD1 au niveau des tissus périphérique et notamment des poumons, pourrait par conséquent produire un effet bénéfique sur la stabilisation puis sa reversion de l'infection.

### H. Hypertrophie cardiaque et insuffisance cardiaque

Les maladies cardiovasculaires représentent la première cause de morbidité et de mortalité dans les pays industrialisés, et l'hypertrophie ventriculaire gauche (HVG) est un facteur de risque indépendant de mortalité cardiovasculaire (Havranek EP, Am J Med 121 :870-875, 2008). En dehors des causes génétiques, des conditions pathologiques telles que l'hypertension artérielle, l'infarctus du myocarde, ou l'insuffisance rénale, peuvent conduire à une hypertrophie compensatoire, progressant par la suite vers une insuffisance cardiaque chronique. L'activité 11βHSD1, permettant la conversion de 11-déshydrocorticostérone en corticostérone, est exprimée dans les cardiomyocytes de rats nouveaux-nés, et contribue à l'activité modulatrice des glucocorticoïdes et de l'aldostérone dans le coeur (Sheppard and Autelitano, Endocrinology 143 :198-204, 2002). En utilisant ces cellules, Lister et al. (Cardiovascular Research 70 : 555-565, 2006) ont montré que l'hypertrophie des cardiomyocytes, induite par des agents pharmacologiques, s'accompagnait d'une augmentation de l'activité de l'enzyme 11βHSD1. Dans cette même étude, l'utilisation du RU-486, antagoniste spécifique des récepteurs aux glucocorticoïdes, permettait de diminuer l'hypertrophie des cellules.

Des inhibiteurs de l'activité 11βHSD1 pourraient donc limiter l'hypertrophie cardiaque et ainsi d'éviter la progression vers l'insuffisance cardiaque.

### I. Maladies hépatiques :

### I1. Stéatose hépatique :

Des études chez des obèses sévères (BMI > 35 kg/m2) rapportent une prévalence de 91 % pour la stéatose et de 37 % pour la stéatohépatite *(*Neuschwander-Tetri & Caldwell, Hepatology, 37, 1202 - 1219, 2003). Le diabète de type 2 est un autre facteur majeur associé à la stéatose avec une prévalence de 70 % rapportée sur un échantillon de 3000 diabétiques italiens (Targher et al., Diabetes Care , 30, 1212 - 1218, 2007). Par ailleurs, il a été observé une association entre l'insulinorésistance et la stéatose hépatique indépendamment de l'obésité chez les patients atteints de stéatose hépatique non alcoolique (Manchesini et al., Diabetes, 50, 1844 - 1850, 2001)*.* Chez les obèses, l'activité 11βHSD1 paraît modifiée ainsi qu'en témoigne l'activation de la cortisone administrée oralement, l'excrétion urinaire des métabolites du cortisol ou l'expression hépatique tissulaire de la 11βHSD1. (Tomlinson et al., Endocrine Rev, 25, 831 - 866, 2004 *;* Rask et al., J. Clin. Endocrin. Metab., 86, 1418 - 1421, 2001 ; Stewart et al., J. Clin. Endocrin. Metabol. 84, 1022-1027, 1999 *;* Valsamakis et al., J. Clin. Endocrinol. Metabol., 89, 4755 - 4761, 2004*)* Les souris transgéniques surexprimant 11 betaHSD1 au niveau du tissu adipeux ou au niveau hépatique développent une stéatose hépatique et une dyslipidémie (Masuzaki et al., Sciences 294, 2166 - 2170, 2001 ; Paterson et al., PNAS, 101, 7088 - 7093, 2004). L'inhibition de la 11βHSD1 chez le rat réduit la triglycéridémie à jeûn suite à une diminution de la sécrétion des triglycérides hépatiques et à une augmentation de la capture et de l'oxydation tissulaire des acides gras, qui se traduit également au niveau hépatique par une réduction significative des triglycérides (Berthiaume et al., Am. J. Physiol. Endocrinol. Metab., 293, 1045 - 1052, 2007). La réduction locale de glucocorticoïde actif par inhibition de l'activité 11βHSD1 est donc envisagée pour diminuer les effets insulinorésistants et lipidiques des glucocorticoïdes et ainsi diminuer la stéatose hépatique.

### I2. Stéatohépatite Métabolique :

La stéatohépatite métabolique représente un stade d'évolution de la stéatose hépatique métabolique chez certaines personnes. Une corrélation est décrite entre le cortisol urinaire, la concentration en cortisol post-dexaméthasone et le grade de nécroinflammation et de fibrose hépatique chez des sujets atteints de stéatohépatite métabolique suggérant l'existence d'un hypercorticolisme subclinique ou locale (Targher et al., Clin. Endocrinol., 64, 337 - 341, 2006). Une correction générale et locale (au niveau centrolobulaire) de l'insulinorésistance ainsi qu'une amélioration de l'oxydation des acides gras hépatiques par inhibition de l'activité 11βHSD1, et aussi la réduction des effets pro-fibrotiques du cortisol sont donc prédictifs d'une amélioration de l'évolution pathologique.

### 13. Régénération hépatique :

Le foie présente une capacité de régénération importante, tout à fait nécessaire en cas d'agressions d'origines infectieuses ou non, en particulier provenant du tractus digestif. Par exemple une apoptose ou une nécrose hépatique peuvent résulter d'une toxicité médicamenteuse, virale, alcoolique, métabolique, cholestatique ou ischémique vasculaire. Les glucocorticoïdes inhibent la prolifération hépatocytaire et la régénération tissulaire hépatique (Tsukamoto & Kojo, Gut, 30, 387 - 390, 1989 *;* Nagy et al., Hepatology, 28, 423 - 429, 1998 *;* Tannuri et al., Pediatr. Transplantation, 12, 73-79, 2008). Une inhibition de l'activité réductase 11βHSD1 pourrait dans ce contexte diminuer les effets locaux négatifs du cortisol sur la régénération hépatique et sont à mettre en ligne avec les effets pro-angiogéniques de ces inhibiteurs et avec leur action positive sur certains facteurs de croissance.

### J. Cicatrisation des plaies cutanées chroniques :

La cicatrisation des plaies chroniques dépend du contexte pathologique sous-jacent qui modifie et désynchronise les étapes physiologiques de la cicatrisation. Dans l'ulcère chronique du diabétique, l'intérêt potentiel des inhibiteurs de 11βHSD1 doit se voir à la fois dans la correction des manifestations du diabète, en tenant compte du rôle pathologique local des corticoïdes endogènes au niveau de la plaie et de l'état d'avancement pathologique. Il existe un certain nombre d'évidences montrant que les corticoïdes endogènes sont directement impliqués dans l'altération de la cicatrisation des plaies chez l'homme et dans des modèles animaux rongeurs (Goforth et al., J. Foot Surgery, 19, 199 - 2002, 1980 ; Dostal et al, Arch. Surg, 125, 636 -640, 1990 ; Bitard, Am. J. Pathology, 152, 547 - 554, 1998). Une production locale de cortisol est prédite par la présence d'une activité réductase 11βHSD1 au niveau endothélial, fibroblastique, cutané chez l'homme et chez les rongeurs (Gong et al., Steroids 73, 1187 -1196, 2008 *; ;* Hammami et al., J. Clin. Endocrinol. Metabol., 73, 326 - 334, 1991 *;* Cooper et al., ENDO 2003 *;* Teelucksingh et al., Lancet, 335, 1060 - 1063, 1990). Le cortisol et autres glucocorticoïdes inhibent la cicatrisation de l'ulcère cutané par de nombreux mécanismes et à différents étapes : altération de la vasomotricité microcirculatoire, inhibition de la phase inflammatoire en particulier sur la synthèse de prostaglandines, de leukotriènes, de cytokines, comme TNFalpha et les productions de IL-1beta, IL-4.... et la signalisation de IFNgamma, augmentation de l'infection, réduction de la motilité cellulaire et de la prolifération des kératinocytes, réduction de l'expression de facteurs pro-angiogéniques comme VEGF, suppression de l'expression de TGFbeta 1 et 2 essentiels dans la production de collagène par les fibroblastes et leur transformation en myofibroblastes, suppression de l'expression de MMP1, 2 , 9 et 10 et induction de TIMP bloquant ainsi le remodelage, promotion de la différentiation terminale épidermique mais inhibition des premiers stades de différentiation, avec pour conséquence une fragilisation de l'épiderme (Bitard, Am. J. Pathology, 152, 547 - 554, 1998 *,* Beer et al., Vitam. Horm., 59, 217 - 239, 2000 *;* Rosen & Miner, Endocrine Review, 26, 452 - 464, 2005*,* Stojadinovic et al., J. Biol. Chem , 282, 4021 - 4034, 2007). A l'inverse et comme attendu, une inhibition de l'activité réductase 11βPHSD1 est décrite pour induire une vasodilatation, un effet pro-angiogénique et anti-infectieux (voir les chapitres correspondants) et dans certaines situations inflammatoires pour produire une exacerbation et une surexpression de facteur de croissance tel que TGF-beta (Zhang et al., J. Immunology, 179, 6325 - 6335, 2007). Des inhibiteurs de 11βHSD1 devraient donc permettre, en agisant ainsi, d'améliorer la cicatrisation des plaies cutanées chroniques.

On a maintenant trouvé des dérivés d'urée de tétrahydroquinoxaline, portant un noyau adamantane, qui modulent l'activité de la 11βHSD1.

La présente invention a pour objet des composés répondant à la formule (I) : 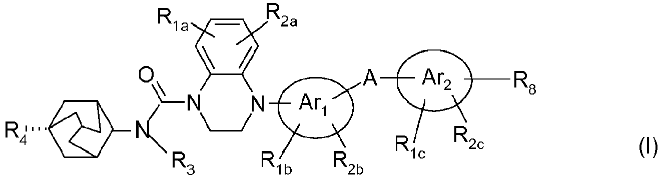 dans laquelle :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c} identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, alcoxy-alcoxy, halogénoalkyle, -O-halogénoalkyle, oxo, -CO-alkyle, -CO-alkyle-NR₆R₇, -CO-halogénoalkyle, -COOR₅, alkyl-COOR₅, -O-alkyl-COOR₅, -SO₂-alkyle, -SO₂-cycloalkyle, -SO₂-alkyl-cycloalkyle, -SO₂-alkyle-OR₅, -SO₂-alkyl-COOR₅, -SO₂-alkyl-NR₆R₇, -SO₂-halogénoalkyle, alkyl-SO₂-alkyle, -SO₂-NR₆R₇, -SO₂-alkyl-alcoxy-alcoxy, -CONR₆R₇, -alkyl-CONR₆R₇ ou -O-alkyl-NR₆R₇, ou encore R₁ₐ, R_{1b}, R_{1c} sont liés respectivement à R₂ₐ, R_{2b}, R_{2c} et à l'atome de carbone qui les porte et représentent -O-alkyl-O- ;
- R₃ représente un atome d'hydrogène ou un groupe alkyle,
- R₄ représente un groupe -CONR₆R₇ ; hydroxy-alkyle substitué par un groupe halogénoalkyle ; -alkyle-NH-SO₂-alkyle ; -NH-SO₂-alkyle ; -O-SO₂-NR₆R₇; -alkyl-CO-NR₆R₇ ; -O-alkyl-CO-NR₆R₇ ; -alkyl-NR₆R₇; -O-CO-NR₆R₇; -alkyl-hétéroaryle ; hétéroaryle éventuellement substitué par un groupe alkyle ; alcoxy-imino ; -CO-NH-NH-CO-alkyle; à la condition que R₄ soit en position cis lorsqu'il représente le groupe -CONR₆R₇ et que R₆ et R₇ représentent chacun l'hydrogène, un groupe -alkyle ou -alkyl-phényle ;
- R₅, représentent l'hydrogène, un groupe alkyle ou un groupe -alkyl-phényle,
- R₆ et R₇, identiques ou différents, représentent chacun un atome d'hydrogène, un groupe alkyle, alcoxy ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un hétéroaryle ou un hétérocycloalkyle éventuellement substitué par 1 à 3 groupes choisis parmi les groupes alkyles, -SO₂-alkyles et -COOR₅.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou d'acides ou être salifiés par des acides ou des bases, notamment des acides ou des bases pharmaceutiquement acceptables. De tels sels d'addition font partie de l'invention. Ces sels sont avantageusement préparés avec des acides ou des bases pharmaceutiquement acceptables, mais les sels d'autres acides ou bases utiles, par exemple, pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules de solvant. De tels solvats font également partie de l'invention.

Dans le cadre de la présente invention, et sauf mention différente dans le texte, on entend par :
- un atome d'halogène : un fluor, un chlore, un brome ou un iode ;
- un groupe alkyle : un groupe aliphatique saturé linéaire ou ramifié comportant de 1 à 5 atomes de carbone. A titre d'exemples, on peut citer les groupes méthyle, éthyle, propyle, méthylpropyle, isopropyle, butyle, isobutyle, tertbutyle ou pentyle ;
- un groupe cycloalkyle : un groupe alkyle cyclique comportant de 3 à 6 atomes de carbone. A titre d'exemples, on peut citer les groupes cyclopropyle, cyclobutyle, cyclopentyle,
- un groupe alcoxy : un radical de formule -O-alkyle, où le groupe alkyle est tel que défini précédemment ;
- un groupe hydroxy-alkyle : un radical de formule alkyle-OH, où le groupe alkyle est tel que défini précédemment ;
- un groupe alcoxy-alkyle : un radical de formule alkyle-O-alkyle, où les groupes alkyles, identiques ou différents, sont tels que définis précédemment. A titre d'exemples, on peut citer -(CH₂)₂-O-CH₃, -(CH₂)₃-O-CH₃, -CH-(CH₂-O-CH₃)₂ ;
- un groupe alcoxy-alcoxy : un radical de formule -O-alkyl-O-alkyle, où les groupes alkyles, identiques ou différents, sont tels que définis précédemment ;
- un groupe halogénoalkyle (abrégé Hal-alkyl): un groupe alkyle tel que défini ci-dessus substitué par 1 à 5 atomes d'halogène, tels que définis précédemment. On citera par exemple le groupe trifluorométhyle ;
- un groupe hétéroaryle : un groupe aromatique comprenant de 5 à 9 atomes, dont 1 à 4 hétéroatomes, tels que l'azote, l'oxygène ou le soufre. On peut notamment citer les groupes pyridinyle, pyrimidinyle, pyridazinyle, thiazolyle, tétrazole, ou oxadiazole ; et
- un hétérocycloalkyle : un groupe alkyle mono-, bi-cyclique éventuellement ponté comportant de 4 à 9 atomes ou éventuellement partiellement insaturé et dont 1 ou 2 atomes sont des hétéroatomes, tels que l'oxygène, l'azote ou le soufre. On peut notamment citer les groupes pyrrolidinyle, pipéridinyle, pipérazinyle, tétrahydropyranyle, morpholinyle, homopipérazinyle, 3,8-diazabicyclo[3.2.1]octane, thiomorpholinyle et thiomorpholinyl-1,1-dioxyde, octahydro-pyrrolo[3,4-c]pyrrole, 1,2,3,6-tetrahydro-pyridine, 2,5-diaza-bicyclo[2.2.1]heptane.
- une fonction carbonyle est représentée par CO.

Dans le cadre de la présente invention, R_{1a,b,c} désigne les groupes R₁ₐ, R_{1b} et R_{1c} et R_{2a,b,c} désigne les groupes R₂ₐ, R_{2b} et R_{2c}. Lorsque Ar₂ représente un groupe hétérocycloalkyle, les groupes R_{1c}, R_{2c} et R₈ peuvent être portés par n'importe quel atome dudit hétérocycle, qu'il s'agisse d'un atome de carbone ou d'un hétéroatome (par exemple un atome d'azote), y compris par le même atome dudit hétérocycloalkyle (par exemple lorsqu'il s'agit d'un atome de soufre).

Dans les composés de formule (I) selon l'invention, le groupe R₄ et le groupe urée peuvent être, sauf stipulation contraire, en position trans ou en position cis. Les composés de formule (I) dans lesquels R₄ et le groupe urée sont en position trans sont particulièrement préférés.

Parmi les composés de formule (I) selon l'invention, on peut citer un sous-groupe de composés dans lesquels A représente une liaison.

Un autre sous-groupe de composés de formule (I) selon l'invention est tel que Ar₁ représente un groupe hétéroaryle. Avantageusement, Ar₁ représente un groupe pyridinyle.

Un autre sous-groupe de composés de formule (I) selon l'invention est tel que Ar₂ représente un groupe hétérocycloalkyle.

Un autre sous-groupe de composés de formule (I) selon l'invention est tel que Ar₂ représente pipérazinyle, homopipérazinyle, 3,8-diazabicyclo[3.2.1]octane, morpholinyle, thiomorpholinyle, octahydro-pyrrolo[3,4-c]pyrrole, 1,2,3,6-tetrahydro-pyridine ou 2,5-diaza-bicyclo[2.2.1]heptane. Avantageusement, Ar₂ représente un groupe pipérazinyle.

Parmi les composés de formule (I) selon l'invention dans lesquels Ar₁ représente un groupe hétéroaryle à 6 chaînons, on peut citer ceux dans lesquels la liaison entre les noyaux A-Ar₂ et Ar₁ est en position para par rapport à la liaison entre Ar₁ et l'atome d'azote du noyau tétrahydroquinoxaline auquel il est lié.

Parmi les composés de formule (I) selon l'invention dans lesquels Ar₂ représente un groupe hétérocycloalkyle, on peut citer ceux qui sont liés au groupe A par un hétéroatome.

Un autre sous-groupe de composés de formule (I) selon l'invention est tel que R₁ₐ, R₂ₐ, R_{1b} et R_{2b}, représentent chacun un atome d'hydrogène.

Un autre sous-groupe de composés de formule (I) selon l'invention est tel que R_{1c} et R_{2c}, identiques ou différents, représentent chacun un atome d'hydrogène ou d'halogène ou un groupe alkyle, ou -SO₂-cycloalkyle.

Un autre sous-groupe de composés de formule (I) selon l'invention est tel que R_{1a,b} et R_{2a,b,c} représentent chacun un atome d'hydrogène et R_{1c} représente l'hydrogène, un groupe alkyle ou -SO₂-cycloalkyle.

Un autre sous-groupe de composés de formule (I) selon l'invention est tel que R₃ représente un atome d'hydrogène.

Un autre sous-groupe de composés de formule (I) selon l'invention est tel que R₈ représente un atome d'hydrogène, ou un groupe de formule -B-Het, où B est absent et Het représente un groupe tétrahydropyranyle.

Les sous-groupes définis ci-dessus pris séparément ou en combinaison font également partie de l'invention.

Un groupe de composés de formule (I) particulierement préférés au sens de l'invention est constitué des composés de formule (I) dans laquelle :
- A est une laison ;
- Ar₁ est un hétéroaryle ;
- Ar₂ est un hétérocycloalkyle ;
- R₃ représente un atome d'hydrogène ou un alkyle,
- R₄ représente un groupe -CONR₆R₇ ; hydroxy-alkyle substitué par un groupe halogénoalkyle ; -alkyle-NH-SO₂-alkyle ; -NH-SO₂-alkyle ; -O-SO₂-NR₆R₇; -alkyl-CO-NR₆R₇; -O-alkyl-CO-NR₆R₇ ; -alkyl-NR₆R₇ ; -O-CO-NR₆R₇; -alkyl-hétéroaryle ; hétéroaryle éventuellement substitué par un groupe alkyle ; alcoxy-imino ; -CO-NH-NH-CO-alkyle; à la condition que R₄ soit en position cis lorsqu'il représente le groupe -CONR₆R₇ et que R₆ et R₇ représentent chacun l'hydrogène, un groupe -alkyle ou -alkyle-phényle ;
- R₅, représentent l'hydrogène, un groupe alkyle ou un groupe -alkyl-phényle,
- R₆ et R₇, identiques ou différents, représentent chacun un atome d'hydrogène, un groupe alkyle, alcoxy ou un groupe -alkyl-phényle, et
- R₈ représente un atome d'hydrogène ; un groupe alkyle ou un groupe tétrahydropyranyle.

Parmi les composés ci-dessus on préfère tout particulièrement ceux dans lesquels R_{1a,b,c} et R_{2a,b,c} sont l'hydrogène et R₈ est un groupe alkyle ou tétrahydropyranyle et ceux dans lesquels R_{1a,b,c} et R_{2a,b} et R₈ sont l'hydrogène et R_{2c} est un groupe alkyle ou -SO₂- cycloalkyle , dans lequel le groupe cycloalkyle est de préférence le groupe cyclopropyle.

Un autre sous-groupe de composés de formule (I) selon l'invention est tel que R₄ représente un groupe :
- CONH₂ ; hydroxyméthyle substitué par un groupe trifluorométhyle ; -CH₂NH-SO₂-Me ; -NH-SO₂-Me ; -CH₂-CO-NH₂ ; -CH₂-NH₂ ; -OCONH₂ ; -CONH-OMe ; tétrazolylméthyle et méthyloxadiazolyle.

Parmi les composés de formule (I) objets de l'invention, on peut citer notamment les composés suivants :
Acide Cis 4-{5-[4-(Tetrahydro-pyran-4-yl)-piperazin-1-yl]-pyridin-2-yl}-3,4-dihydro-2H-quinoxaline-1-carboxylique(5-carbamoyl-adamantan-2-yl)-amide ;
Acide Trans 4-[5-(4-tert-Butyl-piperazin-1-yl)-pyridin-2-yl]-3,4-dihydro-2H-quinoxaline-1-carboxylique[5-(methanesulfonylamino-methyl)-adamantan-2-yl]-amide ;
Acide Trans 4-[5-(4-Cyclopropanesulfonyl-piperazin-1-yl)-pyridin-2-yl]-3,4-dihydro-2H-quinoxaline-1-carboxylixique(5-carbamoylmethyl-adamantan-2-yl)-amide ;
Acide Trans 4-[5-(4-Cyclopropanesulfonyl-piperazin-1-yl)-pyridin-2-yl]-3,4-dihydro-2H-quinoxaline-1-carboxylique(5-aminomethyl-adamantan-2-yl)-amide ;
Acide Trans Carbamique4-({4-[5-(4-cyclopropanesulfonyl-piperazin-1-yl)-pyridin-2-yl]-3,4-dihydro-2H-quinoxaline-1-carbonyl}-amino)-adamantan-1-yl ester ;
Acide Trans 4-[5-(4-tert-Butyl-piperazin-1-yl)-pyridin-2-yl]-3,4-dihydro-2H-quinoxaline-1-carboxylique[5-(2,2,2-trifluoro-1-hydroxy-ethyl)-adamantan-2-yl]-amide ;
Acide Trans 4-[5-(4-tert-Butyl-piperazin-1-yl)-pyridin-2-yl]-3,4-dihydro-2H-quinoxaline-1-carboxylique[5-(2H-tetrazol-5-ylmethyl)-adamantan-2-yl]-amide ;
Acide Trans 4-[5-(4-Cyclopropanesulfonyl-piperazin-1-yl)-pyridin-2-yl]-3,4-dihydro-2H-quinoxaline-1-carboxylique(5-methoxycarbamoyl-adamantan-2-yl)-amide ;
Acide Trans 4-[5-(4-tert-Butyl-piperazin-1-yl)-pyridin-2-yl]-3,4-dihydro-2H-quinoxaline-1-carboxylique(5-methanesulfonylamino-adamantan-2-yl)-amide.
Acide Trans4-[5-(4-Cyclopropanesulfonyl-piperazin-1-yl)-pyridin-2-yl]-3,4-dihydro-2H-quinoxaline-1-carboxylique[5-(3-methyl-[1,2,4]oxadiazol-5-yl)-adamantan-2-yl]-amide

Il est à noter que les composés ci-dessus ont été nommés en nomenclature IUPAC par l'intermédiaire du logiciel AutoNom (Beilstein Informations system).

Dans ce qui suit, on entend par groupe protecteur (GP) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 3rd Edition (John Wiley & Sons, Inc., New York).

On entend par groupe partant (Lg, E, J, V, X, Z), dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, paranitrophényle, etc. Des exemples de groupes partants ainsi que des méthodes pour leur préparation sont donnés dans « Advances in Organic Chemistry », J. March, 3rd Edition, Wiley Interscience, p. 310-316.

Conformément à l'invention, on peut préparer les composés de formule générale (I) selon les procédés ci-après.

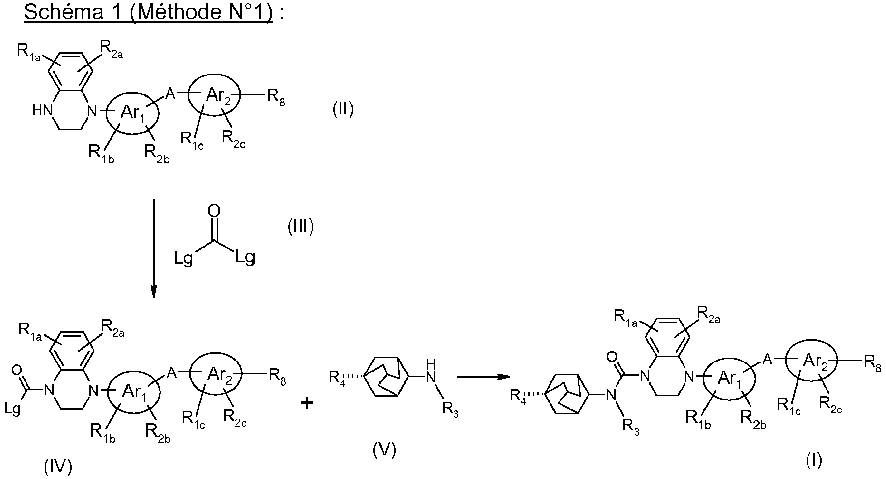

Dans le schéma 1, les composés de formule (IV) peuvent être préparés par réaction entre les intermédiaires de formule (II) et un carbonyle de formule (III) présentant deux groupes partant Lg (par exemple un atome de chlore, un groupe trichlorométhoxy, un groupe para-nitrophényle, un groupe imidazole ou méthyl-imidazolium) en présence d'une base comme la triéthylamine ou la diisopropylamine, dans un solvant tel que le dichlométhane ou le tétrahydrofurane et à une température variant de la température ambiante à 80°C. Les composés de formule (I) sont ensuite obtenus par couplage entre les dérivés activés (IV) et les amines (V) en présence ou non d'une base comme la triéthylamine ou le carbonate de potassium, dans un solvant tel que le tétrahydrofurane, le dichlorométhane, l'acétonitrile, le diméthylformamide ou l'eau, à une température variant de la température ambiante à 100°C.

Dans certains cas, quand R₁ ou R₂ est un alcool, ou R₁ R₂ ou R₄ est une amine primaire ou secondaire ou un acide ou un bioisostère de fonction acide (tétrazole,...) ou si Ar₁ ou Ar₂ présente dans le composé (I) une fonction amine secondaire, il est alors nécessaire de réaliser la Méthode N°1 avec un dérivé (II) ou (V) où les fonctions précédemment citées sont rendues non réactives par la présence d'un groupe protecteur (par exemple, pour une amine : un groupe Boc, Bn ou Cbz ; pour un alcool : un groupe Bn ; pour un acide : un groupe ester ; pour un tétrazole : un groupe benzyle). Finalement, pour obtenir la fonctionnalité désirée, il faut ensuite réaliser une réaction de déprotection dans des conditions connues par l'homme du métier.

Les hétérocycles de formule générale (V) sont disponibles commercialement ou peuvent être préparés par des méthodes décrites dans la littérature (par exemple WO 2007/077949, US 2005/0215784 A1, US 2005/0245745 A1, Journal of Organic Chemistry (2005), 70(20), 7919-7924).

Le schéma 2 détaille une synthèse des composés de formule (I) dans lesquels R₄ représente une fonction aminoalkyle ; ces composés seront appelés ci-après composés de formule (VI).

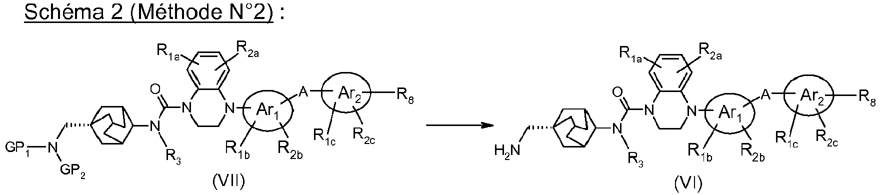

Dans le schéma 2, les composés (VII) présentent une fonction aminoalkyle protégée au moyen de deux groupes protecteurs (GP₁ et GP₂). Les composés (VI) sont obtenus par déprotection de la fonction amine des composés de formule (VII), par exemple si GP₁ et/ou GP₂ est un groupe benzyle ou carboxybenzyle, les méthodes de déprotection possibles comprennent entre autres l'utilisation d'hydrogène en présence d'un catalyseur dérivé du palladium pour réaliser une réaction d'hydrogénolyse, dans un solvant ou mélange de solvants tel que le méthanol, l'éthanol, l'acétate d'éthyle, le tétrahydrofurane, sous une pression d'hydrogène comprise entre 1 et 10 bars à une température variant de la température ambiante à 80°C. Une méthode alternative pour réaliser une hydrogénolyse du groupe protecteur benzyle ou carboxybenzyle consiste à utiliser une catalyse au palladium (par exemple avec du palladium adsorbé sur du charbon) en présence de formiate d'ammonium au reflux d'un solvant tel que le méthanol.

Le schéma 3 détaille une synthèse des composés de formule (I) dans lesquels R₄ représente un groupe hydroxy-alkyle substitué par un halogénoalkyle ; ces composés seront appelés ci-après composés de formule (VIII).

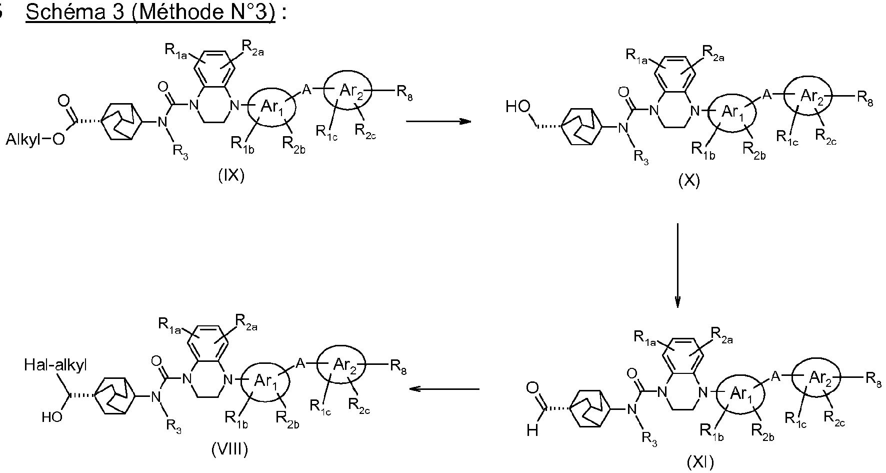

Dans le schéma 3, les dérivés (X) sont obtenus par réduction de la fonction ester du composé (IX) en fonction alcool au moyen d'un hydrure tel que LiAIH₄, LiBH₄, DiBal dans un solvant ou mélange de solvants tel que le tétrahydrofurane, l'éther ou le toluène, à une température variant de -78°C à 40°C. L'étape suivante consiste à oxyder la fonction alcool des dérivés (X) en fonction aldéhyde pour obtenir les composés (XI), au moyen d'un oxydant tel que le réactif de Dess-Martin, le PCC ou le Tpap en présence ou non d'un co-oxydant tel que par exemple le N-oxyde de la N-méthylmorpholine dans un solvant tel que le dichlorométhane, le dichloroéthane en général à température ambiante. Une réaction d'oxydation de Swern telle que décrite dans Synthesis 1990, pp 857-870 peut aussi conduire aux mêmes composés (XI). Finalement, les dérivés (VIII) sont obtenus par addition d'un groupe halogénoalkyle, par exemple un groupe trifluorométhyle sur la fonction aldéhyde des composés (XI) au moyen du réactif de Ruppert ou équivalent en présence ou non d'une source d'ion fluorure telle que le TBAF ou le CsF dans un solvant tel que le THF ou le DMF à une température comprise entre -25°C et la température ambiante.

Le schéma 4 détaille une synthèse des composés de formule (I) dans lesquels R₄ représente une fonction carbamate ; ces composés seront appelés ci-après composés de formule (XII).

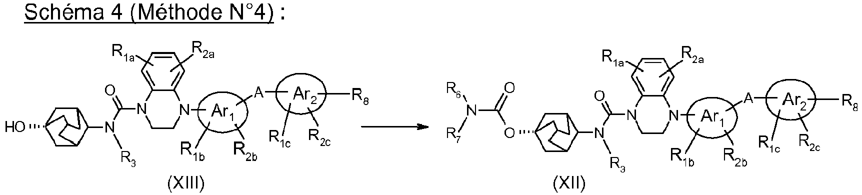

Dans le schéma 4, les dérivés (XII) sont obtenus par une réaction de couplage entre la fonction alcool du dérivé (XIII) et un isocyanate ou un chlorure de carbamoyle en présence ou non d'une base comme la triéthylamine ou le carbonate de potassium, dans un solvant tel que le tétrahydrofurane, le dichlorométhane, l'acétonitrile, le diméthylformamide ou l'eau, à une température variant de la température ambiante à 100°C. Dans le cas particulier où R₆ et R₇ sont deux hydrogènes, le réactif utilisé peut être le trichloroacétylisocyanate et il est nécessaire de retirer ensuite la protection sur l'atome d'azote par une hydrolyse basique avec par exemple K₂CO₃ comme base, dans un solvant tel que le méthanol et à une température comprise entre la température ambiante et 50°C.

Le schéma 5 détaille une synthèse des composés de formule (V) dans lesquels R₃ est un hydrogène et R₄ un groupe alkyl sulfonamide ; ces composés seront appelés ci-après composés de formule (XIV).

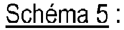

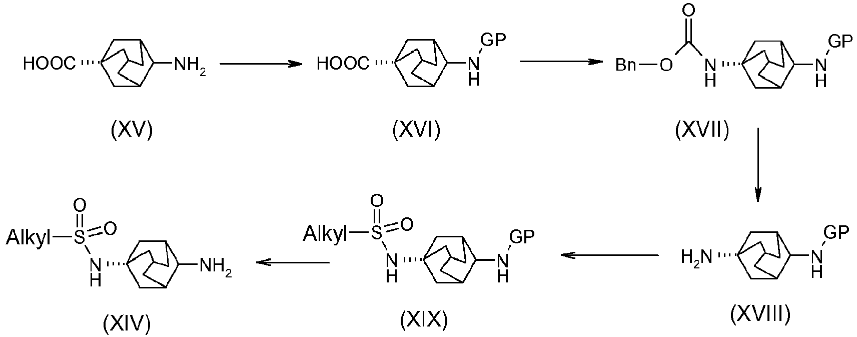

Dans le schéma 5, les dérivés (XVI) sont obtenus par protection de la fonction amine du dérivé (XV), par exemple par un groupe Boc au moyen des méthodes connues par l'homme du métier. Dans une deuxième étape, la fonction acide des dérivés (XVI) subit une réaction de Curtius telle que par exemple décrite avec des dérivés d'adamantane par Maison et al., dans J. Org. Chem. 2008, pp 1056 ou par Broadhurst et al., dans. J. Med. Chem. 2004, pp 4975. Les amines (XVIII) sont obtenues par déprotection de la fonction carbamate des composés de formule (XVII) par des méthodes choisies parmi celles connues de l'homme du métier. Les méthodes de déprotection possibles comprennent entre autres l'utilisation d'hydrogène en présence d'un catalyseur dérivé du palladium pour réaliser une réaction d'hydrogénolyse, dans un solvant ou mélange de solvants tel que le méthanol, l'ethanol, l'acétate d'éthyle, le tétrahydrofurane, sous une pression d'hydrogène comprise entre 1 et 10 bars à une température variant de la température ambiante à 80°C. Une méthode alternative pour réaliser une hydrogénolyse du groupement Cbz consiste à utiliser une catalyse au palladium (par exemple avec du palladium adsorbé sur du charbon) en présence de formiate d'ammonium au reflux d'un solvant tel que le méthanol. Dans l'étape suivante, les composés (XVIII) sont mis en réaction avec un chlorure de sulfonyle en présence d'une base telle que la triéthylamine ou la pyridine dans un solvant tel que le dichorométhane, le choroforme ou le THF à une température comprise entre -10°C et 50°C. Les amines (XIV) sont obtenues par déprotection de la fonction amine des composés de formule (XIX), par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Le schéma 6 détaille une synthèse des composés de formule (V) dans lesquels R₃ est un hydrogène et R₄ un groupe alkyl-sulfonamide-alkyle ; ces composés seront appelés ci-après composés de formule (XX).

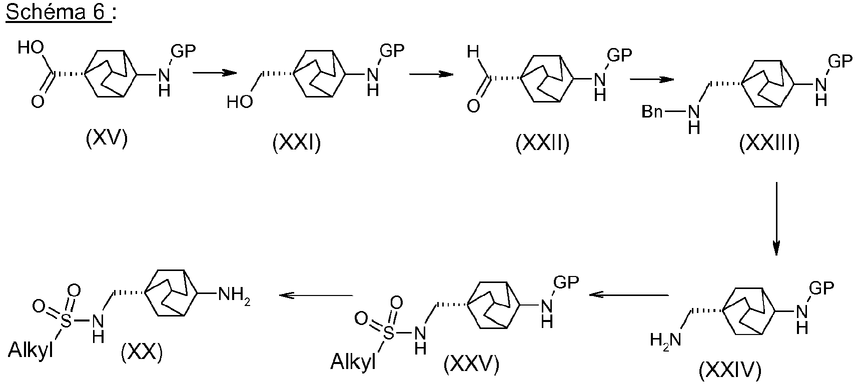

Dans le schéma 6, les dérivés (XXI) sont obtenus par réduction de la fonction acide du composé (XV) en fonction alcool au moyen d'un hydrure tel que LiAlH₄, BH₃,THF dans un solvant ou mélange de solvants tel que le tétrahydrofurane, l'éther ou le toluène, à une température variant de -78°C à 40°C. L'étape suivante consiste à oxyder la fonction alcool des dérivés (XXI) en fonction aldéhyde pour obtenir les composés (XXII), au moyen d'un oxydant tel que le réactif de Dess-Martin, le PCC ou le Tpap en présence ou non d'un co-oxydant tel que par exemple le N-oxyde de la N-méthylmorpholine dans un solvant tel que le dichlorométhane, le dichloroéthane en général à température ambiante. Une réaction d'oxydation de Swern telle que décrite dans Synthesis 1990, pp 857-870 peut aussi conduire aux mêmes composés (XXII). La fonction aldéhyde du dérivé (XXII) est transformée en groupe amine pour obtenir les composés (XXIII) au moyen d'une réaction d'amination réductrice avec une amine telle que la benzylamine en utilisant un réducteur tel que le borohydrure de sodium, le triacétoxyborohydrure de sodium ou le cyanoborohydrure de sodium, en présence ou non d'un acide de Bronsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C. Les dérivés (XXIV) sont ensuite obtenus par déprotection de la fonction benzyle portée sur le groupe aminométhyle des composés (XXIII). Les méthodes de déprotection possibles comprennent entre autres l'utilisation d'hydrogène en présence d'un catalyseur dérivé du palladium pour réaliser une réaction d'hydrogénolyse, dans un solvant ou mélange de solvants tel que le méthanol, l'éthanol, l'acétate d'éthyle, le tétrahydrofurane, sous une pression d'hydrogène comprise entre 1 et 10 bars à une température variant de la température ambiante à 80°C. Une méthode alternative pour réaliser une hydrogénolyse de groupement Bn consiste à utiliser une catalyse au palladium (par exemple avec du palladium adsorbé sur du charbon) en présence de formiate d'ammonium au reflux d'un solvant tel que le méthanol. Dans l'étape suivante, pour obtenir les dérivés sulfonamides (XXV), les composés (XXIV) sont mis en réaction avec un chlorure de sulfonyle en présence d'une base telle que la triéthylamine ou la pyridine dans un solvant tel que le dichorométhane, le choroforme ou le THF à une température comprise entre -10°C et 50°C. Les amines (XX) sont obtenues par déprotection de la fonction amine des composés de formule (XXV) par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Le schéma 7 détaille une synthèse des composés de formule (V) dans lesquels R₃ est un hydrogène et R₄ un groupe alkylamide ; ces composés seront appelés ci-après composés de formule (XXVI).

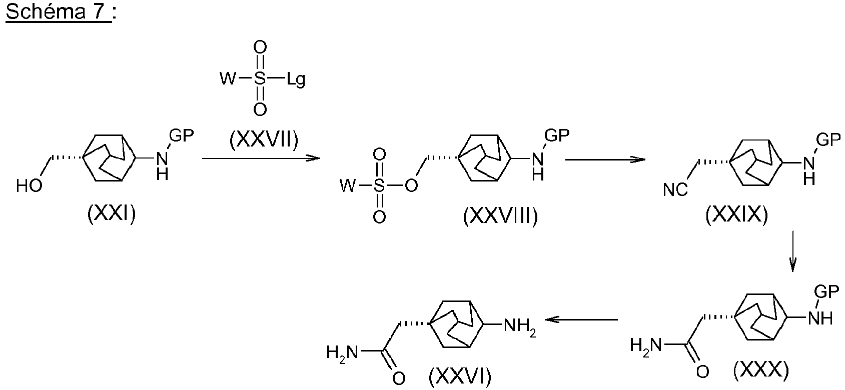

Dans le schéma 7, la fonction alcool des dérivés (XXI) est transformée en ester sulfonique pour former les composés de formule (XXVIII) par action d'un dérivé sulfonique (XXVII) dans lesquels WSO₂ représente par exemple un groupe tosylate, triflate ou nanoflate, tel qu'un anhydride sulfonique (Lg=OSO₂W), un fluorure d'acide sulfonique (Lg=F) ou un chlorure d'acide sulfonique (Lg=Cl), en présence d'une base ou d'un mélange de bases comme la triéthylamine, la pyridine, la diméthylaminopyridine, la diisopropyléthylamine ou le carbonate de potassium dans un solvant ou mélange de solvants tel que le dichlorométhane, le chloroforme, le toluène, le tétrahydrofurane, le diméthylformamide ou l'acétonitrile, à une température variant de -78°C à 100°C. Dans l'étape suivante, l'ester sulfonique des dérivés (XXVIII) est substitué par un groupe cyano pour conduire aux composés (XXIX), au moyen d'un cyanure tel que KCN ou NaCN dans un solvant tel que le DMF ou le DMSO, à une température comprise entre la température ambiante et 150°C. Ensuite, la fonction cyano des dérivés (XXIX) est transformée en groupe amide primaire pour obtenir les composés (XXX) au moyen d'une hydrolyse acide par exemple en utilisant de l'acide sulfurique en absence ou présence d'un solvant comme de l'eau, à une température comprise entre la température ambiante et 100°C. Finalement, dans la dernière étape, les amines (XXVI) sont obtenues par déprotection de la fonction amine des composés de formule (XXX), par des méthodes choisies parmi celles connues de l'homme du métier ; elles comprennent entre autres l'utilisation de pipéridine pour un groupement Fmoc à des températures variant de -10 à 100°C. Dans le cas d'une protection par un groupement Boc, le produit (XXVI) est en général directement obtenu à partir des composés (XXIX) lors de l'hydrolyse acide de la fonction cyano.

Le schéma 8 détaille une synthèse des composés de formule (I) dans lesquels R₄ représente une fonction amide ; ces composés seront appelés ci-après composés de formule (XXXI).

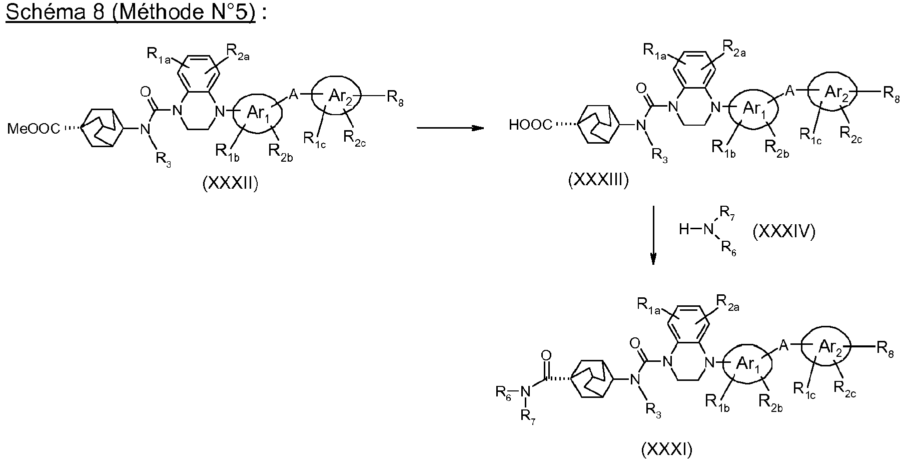

Dans le schéma 8, la fonction ester du composé (XXXII) est saponifiée en fonction acide au moyen de soude, de potasse ou de lithine dans un solvant ou mélange de solvants tel qu'un alcool, de l'eau ou du tétrahydrofurane, à une température variant de la température ambiante à 100°C, pour conduire à l'acide (XXXIII). Dans la dernière étape, le composé (XXXI) peut être préparé par condensation entre l'intermédiaire acide de formule (XXXIII) et une amine (XXXIV) dans des conditions de couplage peptidique classiques, en utilisant par exemple comme agent couplant le dicyclocarbodiimide, le chlorhydrate du 1-(3-diméthylaminopropyl)-3-éthylcarbodiimide ou le bromo-tris-pyrrolidino-phosphonium hexafluorophosphate, en présence ou non d'hydroxybenzo-triazole, et en utilisant comme base organique la triéthylamine ou la diisopropyl-éthylamine dans un solvant ou mélange de solvants tel que le dioxane, le dichlorométhane ou l'acétonitrile.

Le schéma 9 détaille une synthèse des composés de formule (II).

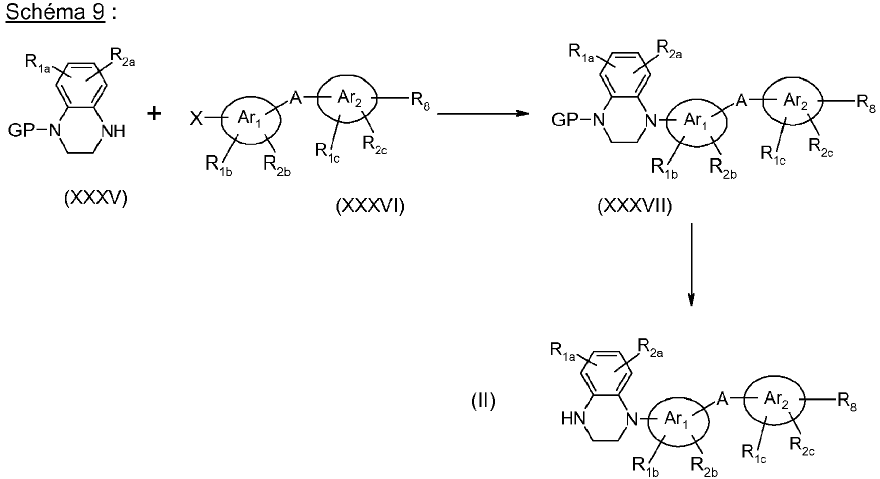

Dans le schéma 9, les composés de formule (XXXVII) peuvent être préparés par couplage entre une tétrahydro-quinoxaline monoprotégée de formule (XXXV) et un dérivé (XXXVI) présentant un groupe partant X (par exemple un halogène, un groupe tosylate, triflate ou nanoflate) en présence d'un catalyseur organométallique tel qu'un dérivé du palladium, en présence ou non d'une phosphine telle que la tritertbutylphosphine ou la triphénylphosphine, en présence d'une base comme le carbonate de potassium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C. Les amines (II) sont obtenues par déprotection de la fonction amine des composés de formule (XXXVII), par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Les hétérocycles de formule générale (XXXV) sont disponibles commercialement ou peuvent être préparés par des méthodes décrites dans la littérature (par exemple «Comprehensive hétérocyclic chemistry », Katritzky et al., 2rd Edition (Pergamon press) ; Krchnak, V. et al., Tet. Lett (2001), 42, 2443-2446 ; Eary, C. T. et al., Tet. Lett. (2006), 47, 6899-6902 ; Savrides, E.-M. et al., J. Het. Chem. (2005), 42, 1031-1034. De Selms, R.C. et al., J. Het. Chem. (1974), 11 (4), 595-7.

Les composés de formule générale (XXXVI) sont disponibles commercialement ou peuvent être préparés par des méthodes décrites dans la littérature (par exemple Z. Sui et coll., Bioorg. Med. Chem. Lett. (2003), 13, 761-765 ; Chopa, A. B. et al., J. Organomet. Chem. (2005), 690(17), 3865-3877 ; Düggeli, M. et al., Org. Biomol. Chem. (2003), 1 (11), 1894-1899 ; Gros, P. et al., J. Org. Chem (2003), 68(5), 2028-2029 ; Bouillon, A. et al., Tet. (2002), 58(14), 2885-2890 ; Balle, T. et al., J. Med. Chem. (2006), 49(11), 3159-3171 ; M. A. Ismail et al., J. Med. Chem. (2006), 49(17), 5324-5332, Gu, Y. G. et al., J. Med. Chem. (2006), 49(13), 3770-3773 ; Serafin, B. et al., Eur. J. Med. Chem. (1977), 12(4), 325-31 ; Schmidt, H.-W. et al., J. Het. Chem. (1987), 24(5), 1305-7 ; Walsh, D. A. et al., J. Med. Chem. (1990), 33(7), 2028-32 ; WO 2005/042521; EP 0 277 725).

Le schéma 10 détaille une synthèse des composés de formule (XXXVII) dans lesquels Ar₁ représente un noyau pyridine (Y=C) ou pyrimidine (Y=N) ; ces composés seront appelés ci-après composés de formule (XXXVIII).

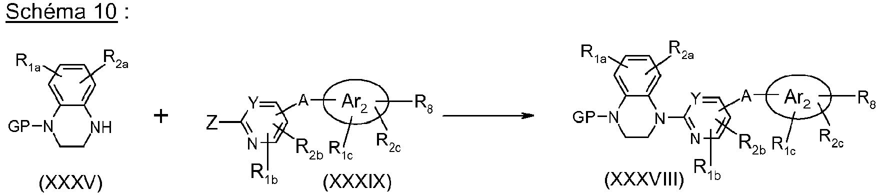

Dans le schéma 10, les composés de formule (XXXVIII) peuvent être préparés par une réaction de substitution nucléophile aromatique entre une tétrahydro-quinoxaline monoprotégée de formule (XXXV) et un dérivé (XXXIX) présentant un groupe partant Z (par exemple un halogène ou un groupe alkylsulfonyle) en présence d'une base comme le sel de lithium de l'hexaméthyldisilazane ou l'hydrure de sodium dans un solvant tel que le tétrahydrofurane, la N-méthylpyrrolidinone, le diméthylsulfoxyde ou le diméthylformamide, à une température variant de la température ambiante à 100°C.

Le schéma 11 détaille une synthèse des composés de formule (XXXVII) dans lesquels Ar₁ représente un noyau phényle et A représente une liaison ; ces composés seront appelés ci-après composés de formule (XXXX).

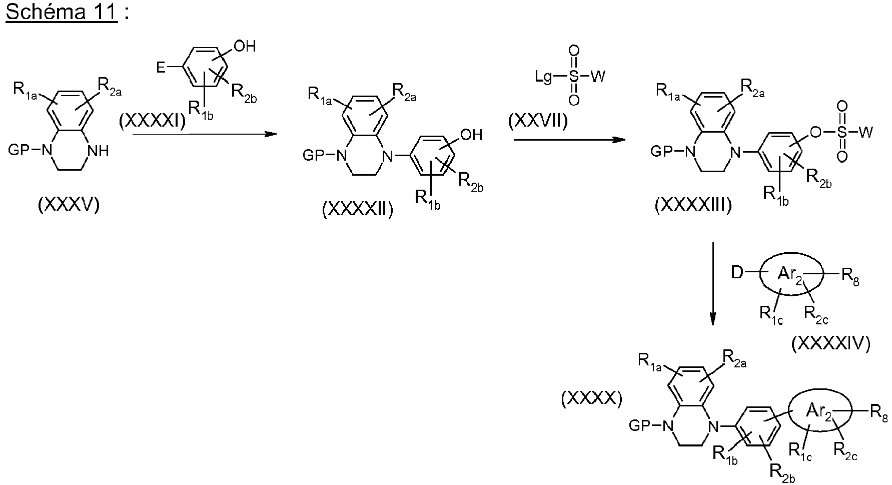

Dans le schéma 11, les composés de formule (XXXXII) peuvent être préparés par une réaction de couplage entre une tétrahydro-quinoxaline monoprotégée de formule (XXXV) et un dérivé (XXXXI) présentant un groupe partant E (par exemple un halogène, triflate ou nanoflate) en présence d'un catalyseur organométallique tel qu'un dérivé du palladium, en présence ou non d'une phosphine telle que la tritertbutylphosphine ou la triphénylphosphine, en présence d'une base comme le carbonate de potassium ou de césium, le fluorure de potassium, le tertbutylate de potassium ou le phosphate de potassium dans un solvant ou mélange de solvants tel que le dioxane, l'éthylène glycol diméthyléther, le toluène, le tétrahydrofurane ou l'eau, à une température variant de la température ambiante à 100°C. La fonction phénol est ensuite transformée en ester sulfonique pour former les composés de formule (XXXXIII) par action d'un dérivé sulfonique (XXVII) où WSO₂ représente par exemple un groupe tosylate, triflate ou nanoflate, tel qu'un anhydride sulfonique (Lg=OSO₂W), un fluorure d'acide sulfonique (Lg=F) ou un chlorure d'acide sulfonique (Lg=Cl), en présence d'une base ou d'un mélange de bases comme la triéthylamine, la pyridine, la diméthylaminopyridine, la diisopropyléthylamine ou le carbonate de potassium dans un solvant ou mélange de solvants tel que le dichlorométhane, le chloroforme, le toluène, le tétrahydrofurane, le diméthylformamide ou l'acétonitrile, à une température variant de -78°C à 100°C. Finalement, les dérivés (XXXX) peuvent être obtenus par une réaction de couplage entre un dérivé (XXXXIII) et un composé (XXXXIV) dans lequel D est soit un groupe organométallique D (par exemple un dérivé du bore, un dérivé de l'étain ou un organozincique) soit un atome d'hydrogène quand il est directement lié à l'atome d'azote d'une amine d'un hétérocycloalkyle, en présence d'une espèce organométallique telle qu'un dérivé du palladium, en présence ou non d'une phosphine telle que la tricyclohexylphosphine ou la triphénylphosphine, en présence d'une base comme le carbonate de potassium ou le fluorure de potassium dans un solvant ou mélange de solvants tel que le dioxane, le diméthylformamide, l'éthylène glycol diméthyléther, le tétrahydrofurane ou l'eau, à une température variant de la température ambiante à 100°C.

Le schéma 12 présente une synthèse alternative des composés de formule (XXXX).

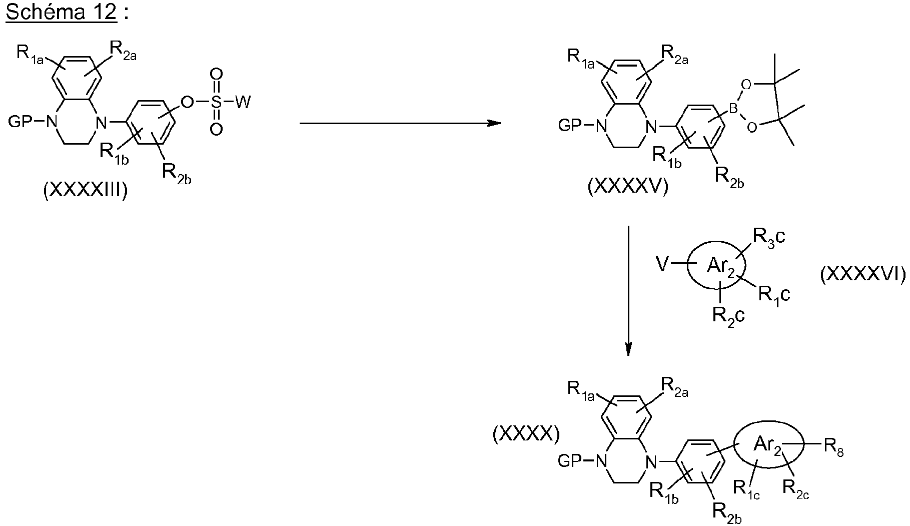

Dans le schéma 12, les composés de formule (XXXXV) peuvent être préparés par transformation de la fonction ester sulfonique des composé (XXXXIII) en une fonction ester boronique pour obtenir les composés de formule (XXXXV) par une réaction avec le bispinacolatodiborane en présence d'un complexe du palladium tel que le 1,1'-bis(diphénylphosphino)ferrocedichloropalladium (II) en présence d'une base comme l'acétate de potassium et de chlorure de lithium dans un solvant ou mélange de solvants tel que le dichlorométhane, le dioxane ou le diméthylsulfoxyde, à une température variant de la température ambiante à 100°C. Dans une deuxieme étape, les dérivés (XXXX) peuvent être obtenus par une réaction de couplage entre le dérivé (XXXXV) et un composé (XXXXVI) présentant un groupe partant V (par exemple un halogène, un triflate, un nonaflate) en présence d'un catalyseur organométallique tel qu'un dérivé du palladium, en présence ou non d'une phosphine telle que la tricyclohexylphosphine ou la triphénylphosphine, en présence d'une base comme le carbonate de sodium ou potassium ou le fluorure de potassium, dans un solvant ou mélange de solvants tel que le dioxane, le diméthylformamide, l'éthylène glycol diméthyléther, le tétrahydrofurane ou l'eau, à une température variant de la température ambiante à 100°C.

Le schéma 13 détaille une synthèse des composés de formule (XXXVII) dans lesquels Ar₁ représente un noyau pyridine (un seul des deux atomes Y est un azote, l'autre est un carbone) et A représente une liaison ; ces composés seront appelés ci-après composés de formule (XXXXVII).

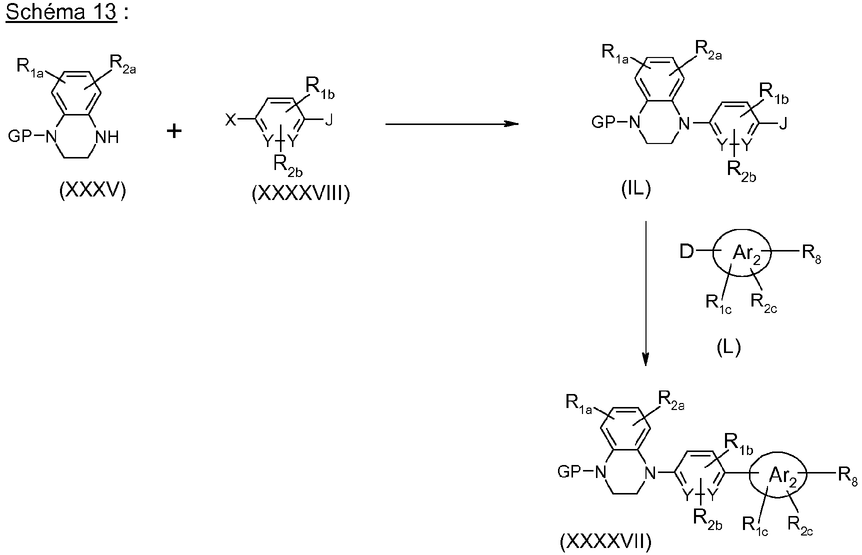

Dans le schéma 13, les composés de formule (IL) peuvent être préparés par une réaction de substitution nucléophile aliphatique ou aromatique entre une tétrahydro-quinoxaline monoprotégée de formule (XXXV) et un dérivé (XXXXVIII) présentant un groupe partant X (par exemple un atome de fluor) et un groupe partant J (par exemple un atome de brome) en présence d'une base comme le tertbutylate de potassium ou l'hydrure de sodium dans un solvant tel que la N-méthylpyrrolidinone ou le diméthylformamide, à une température variant de la température ambiante à 100°C. Finalement, les dérivés (XXXXVII) peuvent être obtenus par une réaction de couplage entre un dérivé (IL) et un composé (L) dans lequel D est soit un groupe organométallique (par exemple un dérivé du bore, un dérivé de l'étain ou un organozincique) soit un atome d'hydrogène quand il est directement lié à l'atome d'azote d'une amine d'un hétérocycloalkyle, en présence d'un catalyseur organométallique tel qu'un dérivé du palladium, en présence ou non d'une phosphine telle que la tricyclohexylphosphine ou la triphénylphosphine, en présence d'une base comme le carbonate de potasium ou cesium, le triphosphate de potassium, le tertbutylate de sodium ou de potassium ou le fluorure de potassium, dans un solvant ou mélange de solvants tel que le toluène, le dioxane, le diméthylformamide, l'éthylène glycol diméthyléther, le tétrahydrofurane ou l'eau, à une température variant de la température ambiante à 100°C.

Le schéma 14 détaille une synthèse des composés de formule (I) dans lesquels R₄ représente un groupe 1,2,4-oxadiazole substitué par un alkyle ; ces composés seront appelés ci-après composés de formule (LI).

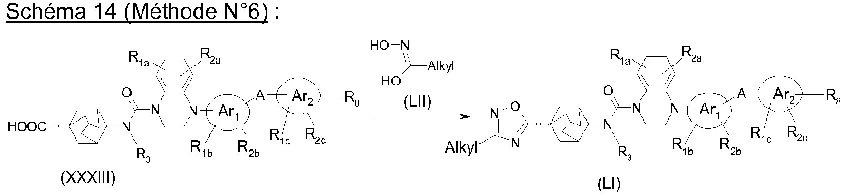

Dans le schéma 14, les composés (LI) sont obtenus par la réaction des acides (XXXIII) avec les dérivés hydroxyamidines (LII) en présence d'un agent de couplage tel que le O-benzotriazol-1-yl-N,N,N',N'-tétraméthyluronium tétrafluoroborate, le DIC, le DCC ou l'EDC, en présence ou non de 1-hydroxybenzotriazole, d'une base comme la triéthylamine, la diisopropyléthylamine dans un solvant tel que le diméthylformamide, le tétrahydrofurane ou l'acétonitrile à une température variant entre la température ambiante et 100°C. Si la réaction de cyclisation n'est pas obtenue, il peut être nécessaire de réaliser une étape supplémentaire de déshydratation cyclisante en présence d'acétate de sodium.

Le schéma 15 détaille une synthèse des composés de formule (I) dans lesquels R₄ représente un groupe alcoxy-imino ; ces composés seront appelés ci-après composés de formule (LIII).

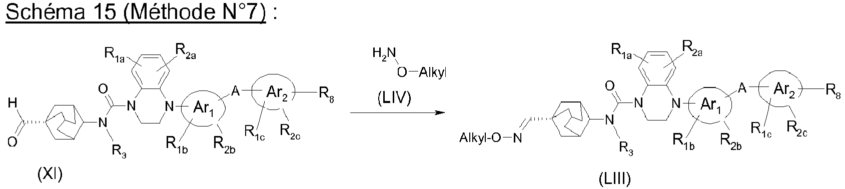

Dans le schéma 15, les oximes (LIII) sont obtenues par la transformation de la fonction aldéhyde des composés (XI) au moyen de O-alkyl-hydroxyamine sous forme de base ou de chlorhydrate en présence ou absence d'une base telle que l'acétate de sodium ou la triéthylamine dans un solvant tel que le méthanol ou l'éthanol à une température variant de 0°C à la température ambiante.

Le schéma 16 détaille une synthèse des composés de formule (V) dans lesquels R₃ est un hydrogène et R₄ un groupe -O-alkyle-CO-NR₆R₇ ; ces composés seront appelés ci-après composés de formule (LV). 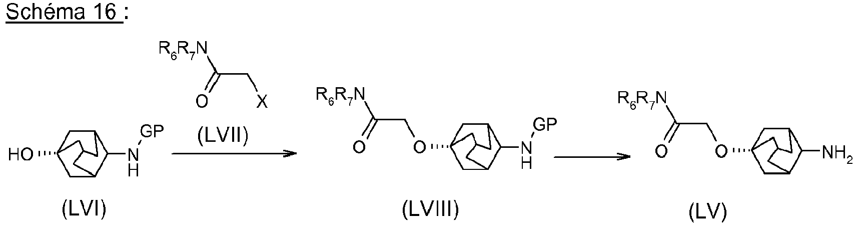 Dans le schéma 16, la fonction alcool des dérivés (LVI) est transformée en groupe -O-alkyle-CO-NR₆R₇ (LVIII) par action d'un dérivé alkylamide (LVII), où X représente un groupe partant, par exemple un atome de brome ou de chlore, un groupe tosylate, triflate ou nanoflate, en présence d'une base ou d'un mélange de bases comme l'hydrure de sodium, le tert-butylate de sodium ou potassium dans un solvant ou mélange de solvants tel que le tétrahydrofurane ou le diméthylformamide, à une température variant de -78°C à 100°C. Finalement, dans l'étape suivante, les amines (LV) sont obtenues par déprotection de la fonction amine des composés de formule (LVIII), par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Le schéma 17 détaille une synthèse des composés de formule (V) dans lesquels R₃ est un hydrogène et R₄ un groupe -O-SO₂-NR₆R₇ ; ces composés seront appelés ci-après composés de formule (LIX).

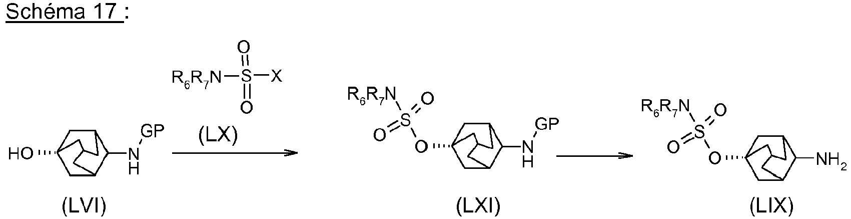

Dans le schéma 17, les dérivés (LXI) sont obtenus par une réaction de couplage entre la fonction alcool du dérivé (LVI) et un chlorure ou bromure de sulfamoyle en présence ou non d'une base comme la triéthylamine ou le carbonate de potassium, dans un solvant tel que le diméthylformamide, le toluène ou l'hexane à une température variant de la température ambiante à 100°C. Finalement, dans l'étape suivante, les amines (LIX) sont obtenues par déprotection de la fonction amine des composés de formule (LXI), par des méthodes choisies parmi celles connues de l'homme du métier ; elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Dans les schémas qui précèdent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

L'invention, selon un autre de ses aspects, a également pour objet les composés de formules (II), (IV), (VII), (IX), (X), (XI), (XIII), (XXXII), (XXXIII), (XXXV), (XXXVI), (XXXVII), (XXXVIII), (XXXX), (XXXXIII), (XXXXV), (XXXXVII), (LV), (LIX) définies ci-avant. Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les abréviations, réactifs usuels et formules brutes suivantes sont utilisées :
- BoC: *tert*-butyloxycarbonyle
- °C: Degré Celsius
- Cbz: Carboxybenzyle
- CsF: Fluorure de césium
- DCC: Dicyclohexylcarbodiimide
- DiBal: Diisobutylaluminium
- DIC: Diisopropylcarbodiimide
- DME: Diméthoxyéthane
- DMF: Diméthylformamide
- DMSO: Diméthyl sulfoxyde
- EDC: 1-Ethyl-3-(3-dimethylaminopropyl)-carbodiimide
- Fmoc: Fluorénylméthoxycarbonyle
- h: heure(s)
- H₂: dihydrogène
- H₂O: eau
- HCl: acide chlorhydrique
- Hobt: Hydroxybenzotriazole
- K₂CO₃: Carbonate de potassium
- LC/MS: chromatographie liquide/ spectrométrie de masse
- ml ou mL: millilitre(s)
- mmol: millimole(s)
- MHz: MegaHertz
- MgSO₄: Sulfate de magnésium
- N: normal
- NMP: N-méthylmorpholine
- NaHCO₃: Hydrogénocarbonate de sodium
- PCC: Chlorochromate de pyridinium
- Pd/C: Palladium sur charbon
- P₂O₅: Pentoxyde de phosphore
- ppm: parties par millions
- psi: pound per square inch
- Reactif de Dess-Martin: (1,1,1-Triacetoxy)-1,1-dihydro-1,2-benziodoxol-3(1H)-one
- Réactif de Ruppert: (Trifluorométhyl)triméthylsilane
- SO₂: dioxyde de soufre
- TBAF: Fluorure de tétrabutylammonium
- Tpap: Perruthénate de tétrapropylammonium
- S-Phos: 2-Dicyclohexylphosphino-2',6'-dimethoxybiphenyl
- Pd₂(dba)₃: tris(dibenzylideneacetone)dipalladium(0)

### Exemple 1 : Trans [5-(Méthanesulfonylamino-méthyl)-adamantan-2-yl]-amide de l'acide 4-[5-(4-tert-butyl-pipérazin-1-yl)-pyridin-2-yl]-3,4-dihydro-2H-quinoxaline-1-carboxylique (composé n°2)

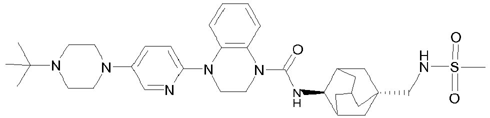

### 1.1 : Trans ester tert-butylique de l'acide (5-hydroxyméthyl-adamantan-2-yl)-carbamique

Dans un ballon tricol sous azote on solubilise 0,5 g de l'acide *trans* 4-tert-butoxycarbonylamino-adamantane-1-carboxylique avec 8,5 ml de tétrahydrofuranne anhydre. La solution est refroidie à 0°C. On ajoute goutte à goutte 5,1 ml d'une solution 1 molaire de complexe borane tétrahydrofurane. On agite 2 h à température ambiante, puis on hydrolyse doucement le milieu réactionnel avec quelques gouttes d'eau. On ajoute une solution aqueuse saturée d'hydrogénosulfate de potassium jusqu'à pH 1 et on extrait le milieu réactionnel à l'acétate d'éthyle jusqu'à épuisement de la phase aqueuse. Les phases organiques sont rassemblées, lavées avec de l'eau puis avec une solution aqueuse saturée de chlorure de sodium, séchées sur sulfate de magnésium et concentrées à sec. On obtient 0,58 g de trans ester tert-butylique de l'acide (5-hydroxymethyl-adamantan-2-yl)-carbamique utilisé tel quel par la suite.
M+H⁺ = 282

### 1.2 : Trans ester tert-butylique de l'acide (5-formyl-adamantan-2-yl)-carbamique

Dans un ballon tricol sous azote on solubilise 0,45 g de trans ester tert-butylique de l'acide (5-hydroxymethyl-adamantan-2-yl)-carbamique avec 16 ml de dichlorométhane anhydre. La solution est refroidie à 0°C. On ajoute 0,75 g de tamis moléculaire 4 angström, puis 0,28 g de 4-méthylmorpholine N-oxyde et 0,028 g de perruthénate de tétrapropylammonium. On agite 4 h à température ambiante. Le milieu réactionnel est filtré sur un mélange composé de 5 g de silice et de 5 g de silice diatomée en rinçant au dichlorométhane. Le filtrat est concentré à sec. On obtient 0,3 g de trans ester tert-butylique de l'acide (5-formyl-adamantan-2-yl)-carbamique utilisé tel quel par la suite.
M+H⁺ = 280

### 1.3 : Trans ester tert-butylique de l'acide [5-(benzylamino-méthyl)-adamantan-2-yl]-carbamique

Dans un ballon tricol sous azote on solubilise 0,2 g de trans ester tert-butylique de l'acide (5-formyl-adamantan-2-yl)-carbamique avec 7 ml de dichlorométhane anhydre. On ajoute 0,084 g de benzylamine, 0,2 g de tamis moléculaire 4 angström, puis 0,23 g de sodium triacétoxyborohydrure. On agite 1 nuit à température ambiante. On rajoute une solution aqueuse 1 N d'acide chlorhydrique et lave une fois avec de l'éther diéthylique. La phase aqueuse est basifiée avec une solution aqueuse 5N de soude puis extraite au dichlorométhane jusqu'à épuisement de la phase aqueuse. Les phases organiques sont rassemblées, lavées avec de l'eau puis avec une solution aqueuse saturée de chlorure de sodium, séchées sur sulfate de magnésium et concentrées à sec. On obtient 0,27 g de trans ester tert-butylique de l'acide [5-(benzylamino-méthyl)-adamantan-2-yl]-carbamique utilisé tel quel par la suite.
M+H⁺ = 371

### 1.4 : Trans ester tert-butylique de l'acide (5-aminométhyl-adamantan-2-yl)-carbamique

Dans un ballon tricol sous azote on solubilise 0,26 g de trans ester tert-butylique de l'acide [5-(benzylamino-méthyl)-adamantan-2-yl]-carbamique avec 7 ml de méthanol. On rajoute 0,062 g de palladium sur charbon à 10% mouillé à 50% avec de l'eau puis 0,45 g de formiate d'ammonium. On chauffe au reflux pendant 1 heure 30 minutes. Le milieu réactionnel est filtré sur un filtre papier. La solution est concentrée à sec. Le residu est repris par une solution aqueuse 1 N de soude. On extrait à l'acétate d'éthyle jusqu'à épuisement de la phase aqueuse. Les phases organiques sont rassemblées, lavées avec de l'eau puis avec une solution aqueuse saturée de chlorure de sodium, séchées sur sulfate de magnésium et concentrées à sec. On obtient 0,2 g de trans ester tert-butylique de l'acide (5-aminométhyl-adamantan-2-yl)-carbamique utilisé tel quel par la suite.
M+H⁺ = 281

### 1.5: Trans ester tert-butylique de l'acide [5-(méthanesulfonylamino-méthyl)-adamantan-2-yl]-carbamique

Dans un ballon tricol sous azote on solubilise 0,2 g de trans ester tert-butylique de l'acide (5-aminométhyl-adamantan-2-yl)-carbamique avec 7 ml de dichlorométhane anhydre. On rajoute 0,12 ml de triéthylamine puis la solution est refroidie à 0°C. 0,06 ml de chlorure de mésyle sont introduits au goutte à goutte. Le milieu est agité 4 heures à température ambiante puis on l'hydrolyse doucement avec de la glace. On extrait à l'acétate d'éthyle jusqu'à épuisement de la phase aqueuse. Les phases organiques sont rassemblées, lavées avec de l'eau puis avec une solution aqueuse saturée de chlorure de sodium, séchées sur sulfate de magnésium et concentrées à sec. Le brut obtenu est chromatographié sur gel de silice en éluant avec un gradient de méthanol dans le dichlorométhane allant de 0 à 5%. On obtient 0,17 g de trans ester tert-butylique de l'acide [5-(méthanesulfonylamino-méthyl)-adamantan-2-yl]-carbamique
M+H⁺ = 359

### 1.6 : Trans N-(4-amino-adamantan-1-ylméthyl)-méthanesulfonamide

Dans un ballon tricol sous azote on solubilise 0,16 g de trans ester tert-butylique de l'acide [5-(méthanesulfonylamino-méthyl)-adamantan-2-yl]-carbamique avec 1 ml de dichlorométhane anhydre. On rajoute 1,7 ml d'une solution 4N d'acide chlorhydrique dans le dioxane. Le milieu réactionnel est agité 4 h à température ambiante puis concentré à sec. On obtient 0,13 g de trans N-(4-amino-adamantan-1-ylméthyl)-méthanesulfonamide utilisé tel quel par la suite.

### 1.7 : Ester tert-butylique de l'acide 4-(5-Bromo-pyridin-2-yl)-3,4-dihydro-2H-quinoxaline-1-carboxylique

On place 30 g de l'ester tert-butylique de l'acide 3,4-dihydro-2H-quinoxaline-1-carboxylique dans 430 ml de N-méthylpyrrolidinone à 0°C sous azote. On ajoute par portion 30 g de tert-butylate de potassium en maintenant la température en dessous de 10°C. On agite 1h30 à température ambiante, puis à 0°C on ajoute 850 ml d'eau et 800 ml d'éther éthylique. La phase aqueuse est extraite par 800 ml d'éther éthylique, puis par 400 ml d'éther éthylique. Les phases organiques sont rassemblées, puis séchées sur sulfate de magnésium et concentrées à sec. On ajoute 300 ml de pentane au brut réactionnel et on sonique sous ultra-sons pendant 5 min le mélange hétérogène obtenu. Le mélange est placé pendant 48h à 5°C, puis le solide est filtré, lavé trois fois au pentane puis séché à 40°C pendant 5h. On obtient 35 g de l'ester tert-butylique de l'acide 4-(5-Bromo-pyridin-2-yl)-3,4-dihydro-2H-quinoxaline-1-carboxylique.
M+H⁺=392.0

### 1.8: Ester tert-butylique de l'acide 4-[5-(4-tert-butyl-pipérazin-1-yl)-pyridin-2-yl]-3,4-dihydro-2H-quinoxaline-1-carboxylique.

On place 28 g de l'ester tert-butylique de l'acide 4-(5-Bromo-pyridin-2-yl)-3,4-dihydro-2H-quinoxaline-1-carboxylique et 9,7 g de 4-tert-butyl pipérazine dans 310 ml de toluène, puis on ajoute 2,5 g de tris(dibenzylidèneacétone)dipalladium (0), 4,48 g de 2-dicyclohéxylphosphino-2',6'-diméthoxybiphényle et 9,17 g de terbutylate de sodium. Le milieu réactionnel est chauffé à 110°C pendant 16 h. On ajoute ensuite de l'acétate d'éthyle et le mélange est lavé une fois à l'eau et une fois avec une solution aqueuse saturée de chlorure de sodium. La phase organique est séchée sur sulfate de magnésium et concentrée sous pression réduite. On obtient 41 g de l'ester tert-butylique de l'acide 4-[5-(4-tert-butyl-pipérazin-1-yl)-pyridin-2-yl]-3,4-dihydro-2H-quinoxaline-1-carboxylique que l'on utilise sans autre forme de purification.
M+H⁺ = 452, 4

### 1.9 : 1-[5-(4-tert-butyl-pipérazin-1-yl)-pyridin-2-yl]-1,2,3,4-tétrahydroquinoxaline

On place 41 g de l'ester tert-butylique de l'acide 4-[5-(4-tert-butyl-pipérazin-1-yl)-pyridin-2-yl]-3,4-dihydro-2H-quinoxaline-1-carboxylique dans un tricol de 1 l sous atmosphère azotée. On ajoute 132 ml de dichlorométhane, on refroidit le mélange réactionnel au bain de glace et on ajoute goutte à goutte 257 ml d'une solution 4 M d'acide chlorhydrique dans le dioxane. On laisse la température remonter doucement jusqu'à la température ambiante puis on agite pendant 2 h. On dilue le mélange réactionnel avec du dichlorométhane, puis on le refroidit au bain de glace et on ajoute une solution saturée de NaHCO₃ jusqu'à pH = 8. On laisse décanter et on extrait la phase aqueuse avec du dichlorométhane. On lave les phases organiques à l'eau, puis on sèche sur Na₂SO₄ les phases organiques réunies, on les filtre sur fritté et on les concentre. Le brut obtenu est chromatographié sur gel de silice en éluant avec un gradient allant de 100 % de dichlorométhane à un mélange de dichlorométhane/ acétate d'éthyle / méthanol / ammoniaque aqueuse concentrée dans les proportions 70/25/5/1 puis avec un mélange de dichlorométhane/méthanol/ammoniaque aqueuse concentrée dans les proportions 90/10/1. On obtient 0,3 g de 1-[5-(4-tert-butyl-pipérazin-1-yl)-pyridin-2-yl]-1,2,3,4-tétrahydroquinoxaline.
M+H⁺ = 351,4

### 1.10 Trans [5-(Méthanesulfonylamino-méthyl)-adamantan-2-yl]-amide de l'acide 4-[5-(4-tert-Butyl-pipérazin-1-yl)-pyridin-2-yl]-3,4-dihydro-2H-quinoxaline-1-carboxylique

Dans un ballon tricol sous azote on solubilise 0,15 g de 1-[5-(4-tert-Butyl-pipérazin-1-yl)-pyridin-2-yl]-1,2,3,4-tétrahydroquinoxaline avec 4 ml de dichlorométhane anhydre. On rajoute 0,12 ml de triéthylamine puis la solution est refroidie à 0°C. On introduit en 2 fois 0,052 g de triphosgène. On agite 3 h à température ambiante, puis on ajoute 1,5 ml de diméthylformamide, 0,19 ml de N-éthyldiisopropylamine et 0,13 g de trans N-(4-amino-adamantan-1-ylméthyl)-méthanesulfonamide. On chauffe à 50°C pendant 1 nuit. Le milieu réactionnel est hydrolysé avec 50 ml d'eau puis on extrait à l'acétate d'éthyle jusqu'à épuisement de la phase aqueuse. Les phases organiques sont rassemblées, lavées avec de l'eau puis avec une solution aqueuse saturée de chlorure de sodium, séchées sur sulfate de magnésium et concentrées à sec. Le brut obtenu est chromatographié sur gel de silice en éluant avec un gradient allant de 100 % de dichlorométhane à un mélange de dichlorométhane/ acétate d'éthyle / méthanol / ammoniaque aqueuse concentrée dans les proportions 70/25/5/1. On obtient 0,18 g de trans [5-(méthanesulfonylamino-méthyl)-adamantan-2-yl]-amide de l'acide 4-[5-(4-tert-butyl-pipérazin-1-yl)-pyridin-2-yl]-3,4-dihydro-2H-quinoxaline-1-carboxylique.
M+H⁺ = 636

### 1.11 : Chlorhydrate du trans [5-(Méthanesulfonylamino-méthyl)-adamantan-2-yl]-amide de l'acide 4-[5-(4-tert-butyl-pipérazin-1-yl)-pyridin-2-yl]-3,4-dihydro-2H-quinoxaline-1-carboxylique

Dans un ballon tricol sous azote on solubilise 0,18 g de trans [5-(méthanesulfonylamino-méthyl)-adamantan-2-yl]-amide de l'acide 4-[5-(4-tert-butyl-pipérazin-1-yl)-pyridin-2-yl]-3,4-dihydro-2H-quinoxaline-1-carboxylique avec 3 ml de dichlorométhane. On rajoute à température ambiante 1,4 ml d'une solution 0.2N d'acide chlorhydrique dans l'éther diéthylique et on agite 15 minutes. Le milieu réactionnel est concentré à sec puis le résidu est repris avec un minimum d'acétate d'éthyle pour obtenir la cristallisation du sel formé. Le solide précipité est essoré, lavé à l'acétate d'éthyle puis séché sous vide sur du pentoxyde de phosphore. On obtient 0,183 g de chlorhydrate du trans [5-(méthanesulfonylamino-méthyl)-adamantan-2-yl]-amide de l'acide 4-[5-(4-tert-butyl-pipérazin-1-yl)-pyridin-2-yl]-3,4-dihydro-2H-quinoxaline-1-carboxylique.
PF=252°C, M+H⁺= 636.
1H NMR (400 MHz, DMSO-d6) δ ppm = 10,28 (m, 1H), 8,09 (m, 1H), 7,48 (d, J = 7,9 Hz, 2H), 7,18 (m, 2H), 6,94 (m, 2H), 6,85 (m, 1H), 6,06 (d, J = 5,8 Hz, 1H), 4,01 à 3,34 (m, 8H), 3,19 (m, 4H), 2,87 (s, 3H), 2,63 (d, J = 6 Hz, 2H), 2,09 à 1,69 (m, 5H), 1,67 à 1,27 (m, 17H)

### Exemple 2 : Trans (5-Carbamoylméthyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique (composé n°3)

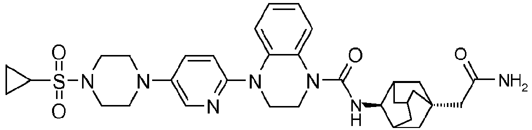

### 2.1 : Trans 2-(4-Amino-adamantan-1-yl)-acétamide

Dans un ballon de 25 ml, on place 0,318 g de trans ester tert-butylique de l'acide (5-cyanométhyl-adamantan-2-yl)-carbamique dans 2ml d'acide sulfurique 95%. On chauffe à reflux pendant 2h. La solution est refroidie à température ambiante. On ajoute 4 ml d'éthanol et on chauffe à reflux pendant 4h. Le milieu réactionnel est versé sur de l'eau plus glace. On évapore la phase aqueuse et on la reprend par du méthanol en mélange avec du dichlorométhane et de l'acétonitrile. On filtre et le filtrat est de nouveau partiellement évaporé. On filtre encore une fois et le filtrat est évaporé à sec, puis repris par du méthanol et séché sur sulfate de magnésium. On obtient 0,259 g d'un mélange 85/15 de trans 2-(4-amino-adamantan-1-yl)-acétamide en mélange avec l'acide trans 2-(4-amino-adamantan-1-yl)-acétique.

### 2.2 : Trans (5-Carbamoylméthyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique

Dans un ballon de 100 ml, on place 0,437 g de 1-[5-(4-cyclopropanesulfonyl-pipérazin-1-yl)-pyridin-2-yl]-1,2,3,4-tétrahydroquinoxaline (intermédiaire 7.5) dans 18 ml d'une solution saturée d'hydrogénocarbonate de sodium. On ajoute 18 ml de dichlorométhane. La solution est refroidie au bain de glace. A +5°C, on ajoute 0,86 ml d'une solution de phosgène à 20% dans le toluène. Au bout de 30 minutes, la réaction est complète. On décante le milieu réactionnel. On ajoute du dichlorométhane à la phase aqueuse et on décante à nouveau. Les phases organiques sont alors réunies, séchées sur sulfate de magnésium, filtrées et évaporées à sec. Ce brut réactionnel est repris dans 10 ml de diméthylformamide. On ajoute 0.95 mL de diisopropyléthylamine. On ajoute 0,259 g d'un mélange 85/15 de trans 2-(4-amino-adamantan-1-yl)-acétamide en mélange avec l'acide trans 2-(4-amino-adamantan-1-yl)-acétique. La solution est agitée à température ambiante pendant deux jours. Le milieu réactionnel est versé sur de l'eau et extrait deux fois à l'acétate d'éthyle. La phase organique est lavée deux fois à l'eau, séchée sur sulfate de magnésium, filtrée et évaporée à sec. Le brut obtenu est chromatographié sur gel de silice en éluant avec un gradient de méthanol dans le dichlorométhane variant de 1% à 10%. On obtient un produit qui est alors trituré dans l'éther éthylique avec quelques gouttes d'acétate d'éthyle. On obtient 0,269 g de trans (5-carbamoylméthyl-adamantan-2-yl)-amide de l'acide 4-[5-(4-Cyclopropanesulfonyl-pipérazin-1-yl)-pyridin-2-yl]-3,4-dihydro-2H-quinoxaline-1-carboxylique.
M+H⁺=634, PF=120-135°C.
1H NMR (400 MHz, DMSO-d6) δ ppm = 8,08 (m, 1H), 7,45 (m, 2H), 7,15 (m, 3H), 6,96 (m, 1 H), 6,88 (m, 1 H), 6,64 (s large, 1 H), 6,03 (d, J = 6,5 Hz, 1 H), 3,81 (m, 4H), 3,69 (m, 1H), 3,47 à 3,13 (m, 8H), 2,68 (m, 1H), 2,07 à 1,31 (m, 15H), 1,01 (m, 4H).

### Exemple 3 : Trans (5-Aminométhyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique (composé n°4)

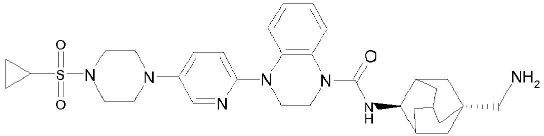

### 3.1 : Ester tert-butylique de l'acide {5-[(benzyl-phénylacétyl-amino)-méthyl]-adamantan-2-yl}-carbamique

Dans un ballon tricol sous azote on solubilise 0,55 g de l'ester tert-butylique de l'acide [5-(benzylamino-méthyl)-adamantan-2-yl]-carbamique avec 1,75 ml de dichlorométhane anhydre. On additionne 0,25 ml de triéthylamine puis 0,25 ml de chloroformiate de benzyle sont introduits goutte à goutte. Le milieu réactionnel est agité pendant 30 minutes à température ambiante puis on rajoute 0,12 ml de triéthylamine et 0,12 ml de chloroformiate de benzyle. La réaction est poursuivie 30 minutes puis on hydrolyse doucement avec de l'eau. On extrait le mélange réactionnel au dichlorométhane jusqu'à épuisement de la phase aqueuse. Les phases organiques sont rassemblées, lavées avec une solution aqueuse saturée d'hydrogénosulfate de potassium, avec une solution aqueuse saturée d'hydrogénocarbonate de sodium, avec une solution aqueuse saturée de chlorure de sodium, puis séchées sur sulfate de magnésium et concentrées à sec. Le brut obtenu est chromatographié sur gel de silice en éluant avec un gradient de méthanol dans le dichlorométhane allant de 0 à 2%. On obtient 0,32 g de l'ester tert-butylique de l'acide {5-[(benzyl-phénylacétyl-amino)-méthyl]-adamantan-2-yl}-carbamique
M+H⁺ = 505

### 3.2 : N-(4-Amino-adamantan-1-ylméthyl)-N-benzyl-2-phényl-acétamide

Dans un ballon tricol sous azote on solubilise 0,49 g de l'ester tert-butylique de l'acide {5-[(Benzyl-phénylacétyl-amino)-méthyl]-adamantan-2-yl}-carbamique avec 1,7 ml de dichlorométhane anhydre. On refroidit jusqu'à 0°C et on ajoute 3,64 ml d'une solution 4N d'acide chlorhydrique dans le dioxane. Le milieu réactionnel est agité 1 h à température ambiante puis concentré à sec. On rajoute 20 ml d'eau, on basifie le mélange réactionnel avec du carbonate de potassium et on l'extrait au dichlorométhane jusqu'à épuisement de la phase aqueuse. Les phases organiques sont rassemblées, lavées avec une solution aqueuse saturée de chlorure de sodium, séchées sur sulfate de magnésium et concentrées à sec. On obtient 0,35g de N-(4-amino-adamantan-1-ylméthyl)-N-benzyl-2-phényl-acétamide utilisé tel quel par la suite.
M+H⁺ = 405

### 3.3 : Trans {5-[(Benzyl-phénylacétyl-amino)-méthyl]-adamantan-2-yl}-amide de l'acide 4-[5-(4-cyclopropanesulfonyl-pipérazin-1-yl)-pyridin-2-yl]-3,4-dihydro-2H-quinoxaline-1-carboxylique

Dans un ballon tricol sous azote on solubilise 0,36 g de1-[5-(4-cyclopropanesulfonyl-pipérazin-1-yl)-pyridin-2-yl]-1,2,3,4-tétrahydro-quinoxaline avec 8,5 ml de dichlorométhane anhydre. On rajoute 0,25 ml de triéthylamine puis la solution est refroidie à 0°C. On introduit en 2 fois 0,106 g de triphosgène. On agite 2 h 30 minutes à température ambiante, puis on ajoute 8,5 ml de diméthylformamide, 0,39 ml de N-éthyldiisopropylamine et 0,35 g de *trans* N-(4-amino-adamantan-1-ylméthyl)-N-benzyl-2-phényl-acétamide. On chauffe à 60°C pendant 1 nuit. Le milieu réactionnel est hydrolysé avec 100 ml d'eau puis on l'extrait à l'acétate d'éthyle jusqu'à épuisement de la phase aqueuse. Les phases organiques sont rassemblées, lavées avec de l'eau puis avec une solution aqueuse saturée de chlorure de sodium, séchées sur sulfate de magnésium et concentrées à sec. Le brut obtenu est chromatographié sur gel de silice en éluant avec un gradient de méthanol dans le dichlorométhane allant de 0 à 5%. On obtient 0,42 g de trans {5-[(benzyl-phénylacétyl-amino)-méthyl]-adamantan-2-yl}-amide de l'acide 4-[5-(4-cyclopropanesulfonyl-pipérazin-1-yl)-pyridin-2-yl]-3,4-dihydro-2H-quinoxaline-1-carboxylique.
M+H⁺ = 831

### 3.4 : (5-Aminométhyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique

Dans un ballon tricol sous azote on solubilise 0,42 g de trans {5-[(benzyl-phénylacétyl-amino)-méthyl]-adamantan-2-yl}-amide de l'acide 4-[5-(4-cyclopropanesulfonyl-pipérazin-1-yl)-pyridin-2-yl]-3,4-dihydro-2H-quinoxaline-1-carboxylique avec 5 ml de méthanol. On rajoute 0,076 g de palladium sur charbon à 10% mouillé à 50% avec de l'eau puis 0,48 g de formiate d'ammonium. On chauffe au reflux pendant 1 heure 30 minutes. Le milieu réactionnel est filtré sur un filtre papier. La solution est concentrée à sec. Le résidu est repris par 50 ml d'eau, on basifie le mélange réactionnel avec du carbonate de potassium et on l'extrait au dichlorométhane jusqu'à épuisement de la phase aqueuse. Les phases organiques sont rassemblées, lavées avec une solution aqueuse saturée de chlorure de sodium, séchées sur sulfate de magnésium et concentrées à sec. Le brut obtenu est chromatographié sur gel de silice en éluant avec un gradient allant de 100 % de dichlorométhane à un mélange de dichlorométhane/ méthanol / ammoniaque aqueuse concentrée dans les proportions 95/5/0,5. On obtient 0,19 g de trans (5-aminométhyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique.
M+H⁺ = 606

### 3.5 : Chlorhydrate du trans (5-aminométhyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique

Dans un ballon tricol sous azote on solubilise 0,18 g de trans (5-aminométhyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique avec 3 ml de dichlorométhane. On rajoute à température ambiante 1,54 ml d'une solution 0.2N d'acide chlorhydrique dans de l'éther diéthylique et on agite pendant 15 minutes. Le milieu réactionnel est concentré à sec puis le residu est repris avec un minimum d'acétate d'éthyle pour obtenir la cristallisation du sel formé. Le solide précipité est essoré, lavé à l'acétate d'éthyle puis séché sous vide sur phosphore pentoxyde. On obtient 0,188 g de chlorhydrate du trans (5-aminométhyl-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique.
M+H⁺= 606, PF=197°C.
1 H NMR (400 MHz, DMSO-d6) δ ppm = 8,04 (d, J = 2,8 Hz, 1 H), 7,88 (m, 3H), 7,49 (m, 2H), 7,17 (m, 2H), 6,94 (m, 2H), 6,14 (d, J = 5,9 Hz, 1H), 4,07 to 3,66 (m, 6H), 3,37 (m, 4H), 3,25 (m, 4H), 2,68 (m, 1H), 2,01 (m, 2H), 1,92 (m, 1H), 1,81 (m, 2H), 1,69 à 1,49 (m, 6H), 1,42 (m, 2H), 1,08 à 0,93 (m, 4H)

### Exemple 4 : Trans ester 4-({4-[5-(4-Cyclopropanesulfonyl-pipérazin-1-yl)-pyridin-2-yl]-3,4-dihydro-2H-quinoxaline-1-carbonyl}-amino)-adamantan-1-ylique de l'acide carbamique (composé n°5)

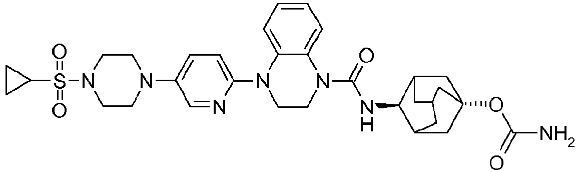

### 4.1 : Trans (5-Hydroxy-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique

Une solution sous azote de 40 mL de dichlorométhane anhydre contenant 1,6 g de 1-[5-(4-cyclopropanesulfonyl-pipérazin-1 -yl)-pyridin-2-yl]-1,2,3,4-tétrahydro-quinoxaline est refroidie à 0°C. 2,23 mL de triéthylamine et 0,475 g de triphosgène sont ensuite ajoutés. Après 1 h 30 d'agitation à température ambiante, 0,816 g de chlorhydrate de trans 4-amino-adamantan-1-ol et 20 mL de diméthylformamide anhydre sont additionnés. L'agitation est maintenue pendant 18 heures. Le milieu réactionnel est chauffé à 35°C pendant 4 heures. Les solvants sont évaporés sous pression réduite. Le résidu est repris par de l'eau. La phase aqueuse est extraite trois fois avec du dichlorométhane. Les phases organiques sont regroupées, lavées trois fois à l'eau, séchées sur sulfate de sodium, filtrées et concentrées à sec. Le résidu obtenu est purifié par chromatographie sur colonne de silice en éluant par un mélange de dichlorométhane/ (dichlorométhane/méthanol 90/10) 90/10 jusqu'à 20/80. On obtient 1,8 g de trans (5-hydroxy-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique.
(M+H⁺)=593

### 4.2 Trans ester 4-({4-[5-(4-Cyclopropanesulfonyl-pipérazin-1-yl)-pyridin-2-yl]-3,4-dihydro-2H-quinoxaline-1-carbonyl}-amino)-adamantan-1-ylique de l'acide carbamique

Une solution sous azote de 1,3 mL de dichlorométhane anhydre contenant 0,15 g de trans (5-hydroxy-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique est refroidie à 0°C. 0,04 mL (1,2 éq) de trichloro-acétyl isocyanate sont additionnés. Après 18 heures d'agitation à température ambiante, le dichlorométhane est évaporé. Le résidu est repris par 1 mL de méthanol et 1,5 mL d'une solution saturée de carbonate de potassium. Après 5 heures de chauffage à 50°C, le milieu réactionnel est refroidi à température ambiante et les solvants sont évaporés. Le résidu est repris par du dichlorométhane. La phase organique est lavée à l'eau, séchée sur sulfate de sodium, filtrée et concentrée à sec à l'évaporateur rotatif. Le résidu obtenu est purifié par chromatographie sur colonne de silice en éluant par un mélange de dichlorométhane/(dichlorométhane/méthanol 90/10) 95/5 jusqu'à 45/55. On obtient 0,07 g de *trans* ester 4-({4-[5-(4-cyclopropanesulfonyl-pipérazin-1-yl)-pyridin-2-yl]-3,4-dihydro-2H-quinoxaline-1-carbonyl}-amino)-adamantan-1-ylique de l'acide carbamique.
M+H⁺=636. PF=décomposition à partir de 106°C.

1 H NMR (400 MHz, DMSO-d6) δ ppm = 8,07 (d, J = 2,9 Hz, 1 H), 7,47 (dd, J = 7,9 Hz et 1,5 Hz, 1H), 7,43 (dd, J = 9 Hz et 3 Hz, 1H), 7,14 (m, 2H), 6,95 (m, 1H), 6,88 (m, 1 H), 6,18 (m, 2H), 6,08 (d, J = 6 Hz, 1 H), 3,80 (m, 4H), 3,76 (m, 1 H), 3,36 (m, 4H), 3,22 (m, 4H), 2,67 (m, 1H), 2,09 (m, 9H), 1,74 (m, 2H), 1,45 (m, 2H), 1,08 à 0.93 (m, 4H)

### Exemple 5 : Trans [5-(2,2,2-trifluoro-1-hydroxy-éthyl)-adamantan-2-yl]-amide de l'acide 4-[5-(4-tert-butyl-pipérazin-1-yl)-pyridin-2-yl]-3,4-dihydro-2H-quinoxaline-1-carboxylique (composé n°6)

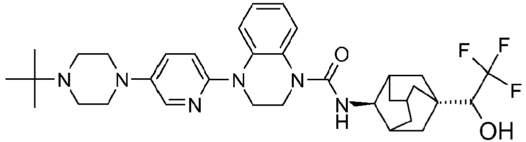

### 5.1 : Ester méthylique de l'acide 4-({4-[5-(4-tert-Butyl-pipérazin-1-yl)-pyridin-2-yl]-3,4-dihydro-2H-quinoxaline-1-carbonyl}-amino)-adamantane-1-carboxylique

Dans un ballon tricol sous atmosphère d'azote on place 1,16 g de 1-[5-(4-tert-butyl-pipérazin-1-yl)-pyridin-2-yl]-1,2,3,4-tétrahydroquinoxaline (intermédiaire 1.9), 33 ml de dichlorométhane et 1,38 ml de triéthylamine. Après refroidissement à -5 °C du milieu par un mélange glace / acétone on ajoute 0,524 g de triphosgène. Le mélange réactionnel est laissé à agiter à température ambiante pendant 3 h. Le milieu réactionnel est concentré sous pression réduite, puis il est repris dans 23 ml de diméthylformamide et 1,38 ml de triéthylamine. On additionne 0,811 g de chorhydrate de methyl ester de l'acide trans 4-amino-adamantan-1-carboxylique. Le milieu réactionnel est laissé sous agitation à température ambiante pendant 18h. Le mélange est dilué par 300 ml de solution saturée de chlorure de sodium et extrait 5 fois par 50 ml d'acétate d'éthyle. Les phases acétate d'éthyle sont regroupées et séchées sur du sulfate de sodium. Après concentration, le brut réactionnel est chromatographié sur gel de silice en éluant avec un gradient de solvants dich!orométhane/méthanol/ammoniaque (100/0/0 à 90/10/1). On obtient 1,15 g de méthyle ester de l'acide 4-({4-[5-(4-tert-butyl-pipérazin-1-yl)-pyridin-2-yl]-3,4-dihydro-2H-quinoxaline-1-carbonyl}-amino)-adamantane-1-carboxylique.
M+H⁺ = 587,2

### 5.2 : Trans (5-hydroxyméthyl-adamantan-2-yl)-amide de l'acide 4-[5-(4-tert-butyl-pipérazin-1-yl)-pyridin-2-yl]-3,4-dihydro-2H-quinoxaline-1-carboxylique

Dans un ballon tricol sous atmosphère d'azote on place 1,14 g de l'ester méthylique de l'acide 4-({4-[5-(4-tert-butyl-pipérazin-1-yl)-pyridin-2-yl]-3,4-dihydro-2H-quinoxaline-1-carbonyl}-amino)-adamantane-1-carboxylique et 19 ml de tétrahydrofuranne anhydre. Après refroidissement à -6 °C du milieu par un mélange glace / acétone on ajoute 2,33 ml d'une solution 1 M de LiAlH₄ dans le tétrahydrofuranne. On laisse la température du milieu réactionnel remonter progressivement jusqu'à 20 °C. Après 3 h le milieu réactionnel est neutralisé par ajout d'une solution à 10 % d'hydrogénosulfate de potassium dans l'eau. Le milieu est ensuite dilué par 150 ml d'eau, alcalinisé par addition d'hydrogénocarbonate de sodium et extrait par du dichlorométhane. La phase dichlorométhane est séchée sur du sulfate de sodium et concentrée sous pression réduite. Le brut obtenu est purifié sur gel de silice en éluant avec un gradient de solvants dichlorométhane/méthanol/ammoniaque (100/0/0 à 90/10/1). On obtient 0,8 g de trans (5-hydroxyméthyl-adamantan-2-yl)-amide de l'acide 4-[5-(4-tert-butyl-pipérazin-1-yl)-pyridin-2-yl]-3,4-dihydro-2H-quinoxaline-1-carboxylique
M+H⁺ =559,2

### 5.3 : Trans (5-Formyl-adamantan-2-yl)-amide de l'acide 4-[5-(4-tert-butyl-pipérazin-1-yl)-pyridin-2-yl]-3,4-dihydro-2H-quinoxaline-1-carboxylique

Dans un ballon tricol sous atmosphère d'azote on place 0,25 g de trans (5-hydroxyméthyl-adamantan-2-yl)-amide de l'acide 4-[5-(4-tert-Butyl-pipérazin-1-yl)-pyridin-2-yl]-3,4-dihydro-2H-quinoxaline-1-carboxylique, 2,5 ml de 1-2 dichloroéthane, 0,12 g de tamis moléculaire 4 angströms et 0,078 g de N-oxyde de N-méthylmorpholine. Le mélange est agité 10 mn à température ambiante puis on additionne 0,008 g de perruthénate de tétrapropylammonium. Le milieu réactionnel est laissé sous agitation à température ambiante pendant 4 h. Après concentration le brut est repris dans de l'acétonitrile, et filtré sur un culot de silice. On obtient 0,16 g de trans (5-formyl-adamantan-2-yl)-amide de l'acide 4-[5-(4-tert-butyl-pipérazin-1-yl)-pyridin-2-yl]-3,4-dihydro-2H-quinoxaline-1-carboxylique.
M+H⁺ = 557,2

### 5.4 : Trans [5-(2,2,2-trifluoro-1-hydroxy-éthyl)-adamantan-2-yl]-amide de l'acide 4-[5-(4-tert-butyl-pipérazin-1-yl)-pyridin-2-yl]-3,4-dihydro-2H-quinoxaline-1-carboxylique

Dans un ballon tricol sous atmosphère d'azote on place 0,16 g de trans (5-formyl-adamantan-2-yl)-amide de l'acide 4-[5-(4-tert-butyl-pipérazin-1-yl)-pyridin-2-yl]-3,4-dihydro-2H-quinoxaline-1-carboxylique et 0,6 ml de tétrahydrofuranne anhydre. Le mélange est refroidi au bain de glace puis on additionne 0,13 ml de trifuorométhyltriméthylsilane. Après quelques minutes d'agitation on ajoute 0,14 ml de solution dans le tétrahydrofuranne 1 M de fluorure de tétrabutylammonium. Le milieu réactionnel est laissé sous agitation jusqu'au lendemain.

Du trifuorométhyltriméthylsilane et du tétrabutylammonium sont régulièrement additionnés sur une période de 48 h jusqu'à ce qu'il n'y ait plus d'évolution de la réaction. Au final les quantités initiales de réactifs ont été doublées. Le milieu réactionnel est dilué par de l'acétate d'éthyle et lavé par de l'eau. Les phases aqueuses sont regroupées et extraites par de l'acétate d'éthyle. Les phases organiques sont ensuite lavées par une solution saturée de chlorure de sodium puis séchées sur du sulfate de sodium. Après concentration sous pression réduite, le brut obtenu est chromatographié sur gel de silice en éluant avec un gradient de solvants dichlorométhane / méthanol (100/0 à 90/10). On obtient 0,015 g de trans [5-(2,2,2-trifluoro-1-hydroxy-éthyl)-adamantan-2-yl]-amide de l'acide 4-[5-(4-tert-butyl-pipérazin-1-yl)-pyridin-2-yl]-3,4-dihydro-2H-quinoxaline-1-carboxylique.
PF=192-196 ; M+H⁺ = 627,2,
1H NMR (400 MHz, DMSO-d6) δ ppm = 8,03 (m, 1H), 7,44 (m, 1H), 7,37 (m, 1H), 7,11 (m, 2H), 6,94 (m, 1 H), 6,86 (m, 1 H), 6,11 (d, J = 7,8 Hz, 1 H), 6,06 (d, J = 6 Hz, 1 H), 3,79 (m, 4H), 3,71 (m, 1H), 3,47 (m, 1H), 3,11 (m, 4H), 2,66 (m, 4H), 1,99 (m, 2H), 1,89 (m, 1 H), 1,83 à 1,63 (m, 8H), 1,44 (m, 2H), 1,06 (m, 9H)

### Exemple 6 : Trans [5-(2H-tétrazol-5-ylméthyl)-adamantan-2-yl]-amide de l'acide 4-[5-(4-tert-butyl-pipérazi n-1-yl)-pyridin-2-yl]-3,4-dihydro-2H-quinoxaline-1-carboxylique (composé n°7)

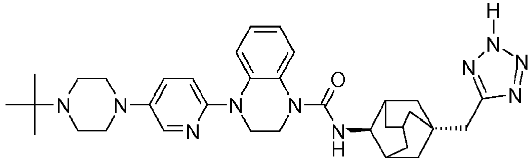

### 6.1 : Trans ester méthylique de l'acide trifluoro-méthanesulfonique acid 4-tert-butoxycarbonylamino-adamantan-1-ylique

4,86 g de trans ester tert-butylique de l'acide (5-hydroxyméthyl-adamantan-2-yl)-carbamique sont dissous dans 88 ml de dichlorométhane. 2,8 ml de pyridine sont additionnés. Le ballon est plongé dans un bain de glace. A +4°C, on ajoute doucement à la seringue 4,1 ml d'anhydride trifluorosulfonique. On laisse agiter à température ambiante pendant 3h. La solution est versée sur de l'eau, extraite deux fois au dichlorométhane. La phase organique est lavée deux fois avec une solution 1N d'acide chlorhydrique, une fois à l'eau et encore une fois avec une solution saturée de chlorure de sodium. Elle est séchée sur du sulfate de sodium, filtrée et évaporée à sec. On obtient 6,29 g de trans méthyle ester de l'acide trifluoro-méthanesulfonique acid 4-tert-butoxycarbonylamino-adamantan-1-ylique en mélange avec 36% de produit de départ.
M-56+ACN+H⁺=399

### 6.2 : Trans ester tert-butylique de l'acide (5-cyanométhyl-adamantan-2-yl)-carbamique

6,29 g de trans méthyle ester de l'acide trifluoro-méthanesulfonic acid 4-tert-butoxycarbonylamino-adamantan-1-ylique sont dissout dans 76 ml de diméthylformamide. On ajoute à température ambiante 1 g de cyanure de potassium. La solution est agitée à température ambiante pendant 2 jours. On verse le milieu réactionnel dans l'eau et on extrait deux fois à l'acétate d'éthyle. La phase organique obtenue est lavée trois fois à l'eau et une fois avec une solution saturée de chlorure de sodium. Elle est séchée sur sulfate de magnésium, filtrée et évaporée à sec. Le brut obtenu est chromatographié sur gel de silice en éluant avec un gradient d'acétate d'éthyle dans l'heptane variant de 0% à 50%. On obtient 1,56 g de trans ester tert-butylique de l'acide (5-cyanométhyl-adamantan-2-yl)-carbamique.
M-56+ACN+H⁺=276

### 6.3 : Trans ester tert-butylique de l'acide [5-(2H-tétrazol-5-ylméthyl)-adamantan-2-yl]-carbamique

0,5 g de trans ester tert-butylique de l'acide (5-cyanométhyl-adamantan-2-yl)-carbamique sont mis en solution dans 8 ml de toluène. On ajoute 0,71 g d'azidotriméthyltétain. On chauffe à reflux pendant 18h. On transvase dans un tube scellé et on chauffe à 120°C pendant 2 jours. On ajoute de nouveau 0,35 g d'azidotriméthyltétain et on chauffe à 130°C pendant 4h puis 18h à 120°C. La solution est versée sur de l'eau, extraite trois fois à l'acétate d'éthyle. La phase organique est lavée à l'eau deux fois, séchée sur sulfate de magnésium, filtrée et évaporée à sec. Le brut obtenu est chromatographié sur gel de silice en éluant avec un gradient de méthanol dans le dichlorométhane variant de 1% à 10%. On obtient 0,367 g de trans ester tert-butylique de l'acide [5-(2H-tétrazol-5-ylméthyl)-adamantan-2-yl]-carbamique.
M-56+ACN+H⁺=319

### 6.4 : Trans ester tert-butylique de l'acide [5-(2-benzyl-2H-tétrazol-5-ylméthyl)-adamantan-2-yl]-carbamique et trans ester tert-butylique de l'acide [5-(1-benzyl-1H-tétrazol-5-ylméthyl)-adamantan-2-yl]-carbamique

On place 0,367 g de trans ester tert-butylique de l'acide [5-(2H-tétrazol-5-ylméthyl)-adamantan-2-yl]-carbamique dans 5,5 ml d'acétone. On ajoute au bain de glace 0,198 g de carbonate de potassium et 0,16 ml de bromure de benzyle. La solution est agitée à température ambiante pendant 2h. On verse sur l'eau et on extrait deux fois à l'acétate d'éthyle. La phase organique est lavée deux fois à l'eau, séchée sur sulfate de magnésium, filtrée et évaporée à sec. Le brut obtenu est chromatographié sur gel de silice en éluant avec un gradient d'acétate d'éthyle dans l'heptane variant de 0% à 30% pendant 45 minutes puis de 30% à 80% pendant 15 minutes. On obtient 0,489 g du mélange de trans ester tert-butylique de l'acide [5-(2-benzyl-2H-tétrazol-5-ylméthyl)-adamantan-2-yl]-carbamique et trans ester tert-butylique de l'acide [5-(1-benzyl-1 H-tétrazol-5-ylméthyl)-adamantan-2-yl]-carbamique.
M+H⁺=424 et 424

### 6.5 : Chlorhydrates du trans 5-(1-benzyl-1 H-tétrazol-5-ylméthyl)-adamantan-2-ylamine et du trans 5-(2-benzyl-2H-tétrazol-5-ylméthyl)-adamantan-2-ylamine

On place 0,466 g du mélange de trans ester tert-butylique de l'acide [5-(2-benzyl-2H-tétrazol-5-ylméthyl)-adamantan-2-yl]-carbamique et de trans ester tert-butylique de l'acide [5-(1-benzyl-1H-tétrazol-5-ylméthyl)-adamantan-2-yl]-carbamique dans 5,5 ml de dichlorométhane. On ajoute à température ambiante 5,5 ml d'acide chlorhydrique 4N dans le dioxane. La solution est agitée pendant 18h. On évapore à sec. On obtient 0,312 g du mélange des chlorhydrates du trans 5-(1-benzyl-1 H-tétrazol-5-ylméthyl)-adamantan-2-ylamine et du trans 5-(2-benzyl-2H-tétrazol-5-ylméthyl)-adamantan-2-ylamine.
M+H⁺=324 et 324

### 6.6 : Trans [5-(2-benzyl-2H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique et trans [5-(1-benzyl-1H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique

Dans un tricol de 50 ml sous atmosphère inerte d'azote, on introduit 0,175 g de 1-[5-(4-tert-butyl-pipérazin-1-yl)-pyridin-2-yl]-1,2,3,4-tétrahydro-quinoxaline (intermédiaire 1.9) dans 5 ml de dichlorométhane. On ajoute à 0°C, 0,21 ml de triéthylamine. On additionne ensuite 0,059 g de triphosgène. Le milieu réactionnel est agité 30 minutes à 0°C puis à température ambiante pendant 3h. 0,24 ml de triéthylamine sont de nouveau ajoutés et 0,2 g de mélange des chlorhydrates du trans 5-(1-benzyl-1 H-tétrazol-5-ylméthyl)-adamantan-2-ylamine et du trans 5-(2-benzyl-2H-tétrazol-5-ylméthyl)-adamantan-2-ylamine sont ensuite additionnés. Pour une meilleure solubilité, on ajoute 5 ml de diméthylformamide. La solution est agitée à température ambiante pendant 3h, puis laissée au repos pendant deux jours. Elle est ensuite versée sur de l'eau et extraite deux fois au dichlorométhane. La phase organique est lavée trois fois à l'eau, séchée sur sulfate de magnésium, filtrée et évaporée à sec. Le brut obtenu est chromatographié sur gel de silice en éluant avec un gradient de méthanol dans le dichlorométhane variant de 1% à 10%. On obtient 0,259 g du mélange de trans [5-(2-benzyl-2H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique et trans [5-(1-benzyl-1H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique.
M+H⁺=701 et 701

### 6.7 : Trans [5-(2H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique

Dans une fiole de Paar de 250 ml, 0,259 g du mélange de trans [5-(2-benzyl-2H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique et du trans [5-(1-benzyl-1H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique sont solubilisés dans 20 ml de méthanol. Sous atmosphère inerte, on ajoute 0,039 g de palladium sur charbon 10%. Au bout de 5h sous 45 psi d'hydrogène à 35°C, puis 1h sous 45 psi à 40°C, la réaction est terminée. La solution est filtrée sur fritté Whatman et évaporée à sec. Le brut obtenu est chromatographié sur gel de silice en éluant avec un gradient de méthanol dans le dichlorométhane variant de 1% à 10% pendant 1 heure et de 10% à 20% pendant 30 minutes. Les cristaux sont triturés dans l'éther éthylique additionné de quelques gouttes d'acétate d'éthyle. Le précipité est alors essoré et séché sous vide à 40°C. On obtient 0,115 g de trans [5-(2H-tétrazol-5-ylméthyl)-adamantan-2-yl]-amide de l'acide 4-[5-(4-tert-butyl-pipérazin-1-yl)-pyridin-2-yl]-3,4-dihydro-2H-quinoxaline-1-carboxylique.
M+H⁺=611, PF=160-170°C.
1H NMR (400 MHz, DMSO-d6) δ ppm = 8,02 (d, J = 3 Hz, 1 H), 7,43 (dd, J = 8 Hz et 1,5 Hz, 1H), 7,36 (dd, J = 9 Hz et 3 Hz, 1H), 7,10 (m, 2H), 6,93 (m, 1H), 6,85 (m, 1H), 6,03 (d, J = 5,9 Hz, 1H), 3,78 (m, 4H), 3,66 (m, 1H), 3,12 (m, 4H), 2,69 (m, 4H), 2,66 (s, 2H), 1,96 (m, 2H), 1,86 (m, 1H), 1,73 (m, 2H), 1,62 à 1,31 (m, 9H), 1,08 (s, 9H)

### Exemple 7: Trans (5-méthoxycarbamoyle-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique (composé n°8)

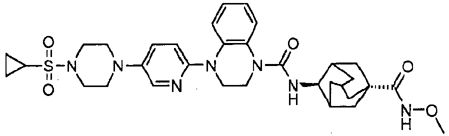

### 7.1 : Ester tert-butylique de l'acide 4-(5-bromo-pyridin-2-yl)-3,4-dihydro-2H-quinoxaline-1-carboxylique.

On place 16 g de l'ester tert-butylique de l'acide 3,4-dihydro-2H-quinoxaline-1-carboxylique dans un tricol de 11, sous atmosphère d'azote. On ajoute 325 ml de pyrrolidinone et 14,70 ml de 5-bromo-2-fluropyridine. On refroidit au bain de glace. On ajoute portion par portion 15,93 g de *tert*-butylate de potassium. On laisse le mélange réactionnel sous agitation à froid pendant 1 h, puis on laisse le milieu réactionnel remonter jusqu'à la température ambiante. On agite pendant 1h. On refroidit au bain de glace et on hydrolyse lentement. La phase organique est extraite à l'éther éthylique. Les phases organiques réunies sont lavées à l'eau, puis avec une solution saturée de NaCl. On sèche le mélange réactionnel sous agitation sur Na₂SO₄, puis on le filtre sur fritté et on le concentre sous pression réduite. On obtient 10,1 g de l'ester tert-butylique de l'acide 4-(5-bromo-pyridin-2-yl)-3,4-dihydro-2H-quinoxaline-1-carboxylique sous forme d'un solide blanc, après purification sur colonne de silice (Gradient Heptane/AcOEt : 5 à 20% en AcOEt).
M+H⁺= 392

### 7.2 : Ester tert-butylique de l'acide 4-cyclopropanesulfonyl-pipérazin-1-carboxylique.

On place 8 g de l'ester tert-butylique de l'acide pipérazine-1-carboxylique dans un tricol de 11, sous atmosphère azotée. On ajoute 330 ml de dichlorométhane, puis 8,9 ml de triéthylamine. On refroidit le mélange réactionnel au bain de glace. On y ajoute goutte à goutte 5,25 ml de chlorure de cyclopropanesulfonyle. On laisse la température remonter doucement jusqu'à température ambiante puis on laisse le mélange réactionnel sous agitation pendant 4h30. On dilue le mélange réactionnel avec du dichlorométhane. Les phases organiques réunies sont lavées à l'eau, puis avec une solution saturée de NaCl, puis ensuite séchées sur Na₂SO₄, filtrées sur fritté et concentrées sous pression réduite. On obtient 13,94 g de ester tert-butylique de l'acide 4-cyclopropanesulfonyl-pipérazin-1-carboxylique sous forme d'une poudre blanche.
M+H (-Boc) = 191

### 7.3 : Chlorhydrate de 1-cyclopropanesulfonyl-pipérazine

On place 14,37 g de ester tert-butylique de l'acide 4-cyclopropanesulfonyl-pipérazine-1-carboxylique dans un tricol de 2 l, sous atmosphère azotée. On ajoute 412 ml de dichlorométhane. On refroidit le milieu réactionnel au bain de glace et on additionne goutte à goutte l'acide chlorhydrique 4 M en solution dans du dioxane. On laisse le milieu réactionnel remonter doucement jusqu'à la température ambiante. Le mélange réactionnel est agité pendant 2h30, puis évaporé et concentré. Le résidu est repris avec une faible quantité d'éther éthylique, trituré puis filtré et séché. On obtient 10,25g de chlorhydrate de 1-cyclopropanesulfonyl-pipérazine sous forme d'une poudre blanche.

### 7.4 : Ester tert-butylique de l'acide 4-[5-(4-cyclopropanesulfonyl-pipérazin-1-yl)-pyridin-2-yl]-3,4-dihydro-2H-quinoxaline-1carboxylique.

On place 11,42 g (29,26 mmol, 1 éq) de l'ester tert-butylique de l'acide 4-(5-bromo-pyridin-2-yl)-3,4-dihydro-2H-quinoxaline-1-carboxylique dans un tricol de 1 l, sous atmosphère azotée. On ajoute 146 ml de toluène anhydre. On additionne 7,63 g (33,65 mmol, 1,15 éq) de chlorhydrate de 1-cyclopropanesulfonyl-pipérazine puis 8,43 g (87,78 mmol, 3 éq) de NaOtBu, 1,92 g (4,68 mmol, 0,16 éq) de S-Phos puis 1,07 g (1,17 mmol, 0,04 éq) de Pd₂(dba)₃. On chauffe le milieu réactionnel à 115°C pendant 1h40. On laisse la température du milieu réactionnel revenir doucement jusqu'à la température ambiante. On filtre sur célite, on concentre, puis on lave le filtrat à l'eau puis avec une solution saturée de NaCl. On sèche sur Na₂SO₄, filtre sur fritté et concentre. Le produit souhaité est obtenu sous forme de poudre blanche, après purification sur colonne de silice (Gradient cyclohexane/AcOEt : 30 à 75% en AcOEt) avec un rendement de 70 % (m = 10,27 g).
M+H⁺ = 501

### 7.5 : 1-[5-(4-cyclopropanesulfonyl-pipérazin-1-yl)-pyridin-2-yl]-1,2,3,4-tétrahydro quinoxaline

On place 6,14 g de l'ester tert-butylique de l'acide 4-[5-(4-cyclopropanesulfonyl-pipérazin-1-yl)-pyridin-2-yl]-3,4-dihydro-2H-quinoxaline-1 carboxylique dans un tricol de 1 l sous atmosphère azotée. On ajoute 102 ml de dichlorométhane, on refroidit au bain de glace et on additionne goutte à goutte 92 ml d'une solution à 4 M d'acide chlorhydrique dans le dioxane. On laisse la température du milieu réactionnel revenir doucement jusqu'à la température ambiante puis on agite pendant 5 h. Le milieu réactionnel est dilué avec du dichlorométhane, puis refroidi au bain de glace. On ajoute ensuite une solution saturée de NaHCO₃ jusqu'à pH = 8. On décante et extrait la phase aqueuse avec du dichlorométhane. Les phases organiques à l'eau sont lavées, séchées sur Na₂SO₄, filtrées sur fritté et concentrées. On obtient 4,92 g de 1-[5-(4-cyclopropanesulfonyl-pipérazin-1-yl)-pyridin-2-yl]-1,2,3,4-tétrahydro-quinoxaline sous forme d'un solide blanc.
M+H⁺ = 400

### 7.6 : Chlorhydrate de trans ester méthylique de l'acide 4-amino-adamantan-1-carboxylique.

On place 2,65 g de l'acide *trans* 4-amino-adamantan-1-carboxylique dans un tricol de 1 l, sous atmosphère azotée. On ajoute 425 ml de méthanol, puis on refroidit au bain de glace. On additionne goutte à goutte 1,10 ml de chlorure de thionyle. Lorsque le milieu réactionnel est limpide, on retire le bain de glace et on chauffe le milieu réactionnel à reflux pendant 3h30. On laisse la température du milieu réactionnel revenir doucement jusqu'à la température ambiante. On le concentre puis on le triture dans l'éther éthylique et quelques ml d'éthanol, on filtre sur fritté, on concentre puis on sèche le produit obtenu. On obtient 1,6 g chlorhydrate de *trans* ester méthylique de l'acide 4-amino-adamantan-1-carboxylique sous forme d'un solide blanc.
M+H⁺ = 246

### 7.7 : Trans ester méthylique de l'acide 4-({4-[5-(4-cyclopropanesulfonyl-pipérazin-1-yl)-pyridin-2-yl]-3,4-dihydro-2H-quinoxaline-carboxyl}-amino)-adamantan-1-carboxylique.

On place 2 g de 1-[5-(4-cyclopropanesulfonyl-pipérazin-1-yl)-pyridin-2-yl]-1,2,3,4-tétrahydroquinoxaline dans un tricol de 500 ml, sous atmosphère azotée. On ajoute 50 ml de dichlorométhane et 2,09 ml de triéthylamine. On refroidit le milieu réactionnel au bain de glace, puis on additionne 0,74 g de triphosgène. On laisse la température du milieu réactionnel remonter doucement jusqu'à la température ambiante. On agite pendant 1h30. On additionne ensuite 1,59 g du *trans* N-4-méthyl-amino-adamantane-1-carboxylate sous forme de chlorhydrate solubilisé à chaud dans 50 ml de DMF en présence de 2,09 ml de triéthylamine. On agite le milieu réactionnel à température ambiante pendant 1h20. Le milieu réactionnel est concentré puis repris par de l'AcOEt. La phase organique est lavée à l'eau, puis séchée sur Na₂SO₄, filtrée sur fritté et concentrée. On obtient 2,74g de *trans* ester méthylique de l'acide 4-({4-[5-(4-cyclopropanesulfonyl-pipérazin-1-yl)-pyridin-2-yl]-3,4-dihydro-2H-quinoxaline-carboxyl}-amino)-adamantan-1-carboxylique sous forme d'une poudre blanche, après purification sur colonne de silice (Gradient dichlorométhane/ CH₂Cl₂/AcOEt/MeOH 75/25/5 : 5 à 80% en CH₂Cl₂/AcOEt/MeOH 75/25/5).
M+H⁺ = 635

### 7.8 : Acide trans 4-({4-(5-(4-cyclopropanesulfonyl-pipérazin-1-yl)-pyridin-2-yl)-3,4-dihydro-2H-quinoxaline-1-carbonyl}-amino)adamantan-1-carboxylique.

On place 2,92 g de *trans* ester méthylique de l'acide 4-({4-[5-(4-cyclopropanesulfonyl-pipérazin-1-yl)-pyridin-2-yl]-3,4-dihydro-2H-quinoxaline-carboxyl}-amino)-adamantan-1-carboxylique dans un tricol de 500 ml, sous atmosphère azotée. On ajoute 23 ml de THF et 11,5 ml de méthanol. On refroidit au bain de glace. On additionne 335 mg d'hydroxyde de lithium en solution dans 11,5 ml d'eau. On laisse le milieu réactionnel remonter doucement jusqu'à la température ambiante. Le milieu réactionnel est ensuite agité pendant 23h puis concentré. On ajoute ensuite de l'eau puis puis du SO₂ jusqu'à pH = 4-5. Le précipité obtenu est trituré avec une faible quantité d'eau, filtré et séché. On obtient 2,71 g de l'acide trans 4-({4-(5-(4-cyclopropanesulfonyl-pipérazin-1-yl)-pyridin-2-yl)-3,4-dihydro-2H-quinoxaline-1-carbonyl}-amino)adamantan-1-carboxylique sous forme d'un solide blanc.
M+H⁺ = 621

### 7.9: trans (5-Méthoxycarbamoyle-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique.

On place 0,40 g de l'acide *trans* 4-({4-(5-(4-cyclopropanesulfonyl-pipérazin-1-yl)-pyridin-2-yl)-3,4-dihydro-2H-quinoxaline-1-carbonyl}-amino)adamantan-1-carboxylique dans un ballon de 50 ml, sous atmosphère azotée. On ajoute 4 ml de dichlorométhane et 3 ml d'acétonitrile. On additionne 0,10 g de chlorhydrate de méthoxyamine, 0,24 g de EDC, 0,19 g de HOBt et 0,72 ml de triéthylamine. On agite le milieu réactionnel à température ambiante pendant 2 jours. On concentre le milieu réactionnel, puis on le reprend par du dichlorométhane ; la phase organique est lavée à l'eau, séchée sur Na₂SO₄, filtrée sur fritté et concentrée. Après purification sur colonne de silice (Gradient : dichlorométhane/CH₂Cl₂/MeOH/NH₄OH 80/20/2 : 5 à 45% en CH₂Cl₂/MeOH/NH₄OH 80/20/2), puis trituration dans l'éther éthylique, filtration et séchage, on obtient 0,22g de *trans* (5-Méthoxycarbamoyle-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique sous forme d'une poudre blanche.
M+H⁺ = 650, PF : 125-163°C.
1H NMR (400 MHz, DMSO-d6) δ ppm = 10,78 (s, 1H), 8,08 (d, J = 3 Hz, 1H), 7,47 (dd, J = 8 Hz and 1,5 Hz, 1H), 7,43 (dd, J = 9 Hz et 3 Hz, 1H), 7,14 (m, 2H), 6,95 (m, 1H), 6,88 (m, 1H), 6,09 (d, J = 6 Hz, 1H), 3,81 (m, 4H), 3,73 (m, 1H), 3,56 (s, 3H), 3,36 (m, 4H), 3,22 (m, 4H), 2,67 (m, 1 H), 2,00 (m, 2H), 1,93 à 1,70 (m, 9H), 1,45 (m, 2H), 1,08 à 0,93 (m, 4H)

### Exemple 8 : Trans (5-méthanesulfonylamino-adamantan-2-yl)-amide de l'acide 4-[5-(4-tert-butyl-pipérazin-1-yl)-pyridin-2-yl]-3,4-dihydro-2H-quinoxaline-1-carboxylique (composé n°9)

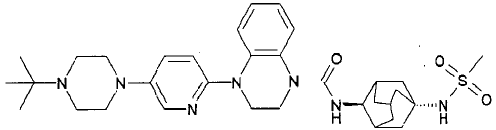

### 8.1 : Trans ester tert-butylique de l'acide (5-amino-adamantan-2-yl)-carbamique

On place 0,7g de *trans* ester benzylique (4-tert-butoxycarbonylamino-adamantan-1-yl)-carbamique dans 18 ml de méthanol puis on ajoute 0,44g de formiate d'ammonium et 0,372g de palladium sur charbon 10% (50% humide). Le milieu réactionnel est mis au reflux pendant deux heures. On filtre ensuite le palladium que l'on rince trois fois au méthanol et on concentre le mélange. On obtient 0,473g de *trans* ester tert-butylique de l'acide (5-amino-adamantan-2-yl)-carbamique qui est utilisé sans autre forme de purification.
M+H+=267

### 8.2 : trans ester tert-butylique de l'acide (5-méthanesulfonylamino-adamantan-2-yl)-carbamique

On place 0,45g de *trans* ester tert-butylique de l'acide (5-amino-adamantan-2-yl)-carbamique dans 8,45 ml de dichlorométhane, puis on ajoute 0,28 ml de triéthylamine et 0,14 ml de chlorure de méthane sulfonyle. Le milieu réactionnel est ensuite agité à température ambiante pendant 18 heures, puis on ajoute au milieu, de l'eau, du dichlorométhane et une solution saturée de chlorure de soduim ; on extrait trois fois la phase aqueuse au dichlorométhane. Les phases organiques sont rassemblées, puis séchées sur du sulfate de magnésium et concentrées sous vide. Le brut obtenu est chromatographié sur gel de silice en éluant avec un gradient d'un mélange heptane/acétate d'éthyle (0 à 50/50). On obtient 0,5g de *trans* ester tert-butylique de l'acide (5-méthanesulfonylamino-adamantan-2-yl)-carbamique.
M+H+=289

### 8.3 : Trans N-(4-Amino-adamantan-1-yl)-méthanesulfonamide

On agite 0,495g de *trans* ester tert-butylique de l'acide (5-méthanesulfonylamino-adamantan-2-yl)-carbamique avec 5,39 ml d'acide chlorhydrique 4N dans le dioxane pendant vingt minutes. On concentre sous pression réduite, on obtient 0,395g de *trans* N-(4-amino-adamantan-1-yl)-méthanesulfonamide brut qui est utilisé sans autre forme de purification.

### 8.4 : Trans (5-méthanesulfonylamino-adamantan-2-yl)-amide de l'acide 4-[5-(4-tert-butyl-pipérazin-1-yl)-pyridin-2-yl]-3,4-dihydro-2H-quinoxaline-1-carboxylique

On place 0,430 g de 1-[5-(4-tert-butyl-pipérazin-1-yl)-pyridin-2-yl]-1,2,3,4-tétrahydro-quinoxaline dans 12,23 ml de dichloromethane et 0,51 ml de triéthylamine, puis on ajoute à 0°C, 0,181g de triphosgène. On agite le mélange réactionnel à température ambiante sous atmosphère d'azote pendant 15 minutes pour former le 4-[5-(4-tert-butyl-pipérazin-1-yl)-pyridin-2-yl]-3,4-dihydro-2H-quinoxaline-1-carbonyl chlorure. On prépare 0.378 g de *trans* N-(4-amino-adamantan-1-yl)-méthanesulfonamide dans 10ml de diméthyle formamide et 0,51 ml de triéthylamine sous atmosphère d'azote que l'on chauffe pour solubiliser. On ajoute le mélange chaud au milieu réactionnel contenant le 4-[5-(4-tert-butyl-pipérazin-1-yl)-pyridin-2-yl]-3,4-dihydro-2H-quinoxaline-1-carbonyl chlorure et on agite pendant deux heures. On ajoute de l'eau, du chlorure de sodium et on extrait trois fois au dichlorométhane ; les phases organiques sont réunies puis séchées sur du sulfate de magnésium, puis concentrées sous pression réduite. Les 0,829 g de brut sont chromatographiés sur gel de silice en éluant avec un gradient d'un mélange dichlorométhane/(dichlorométhane- méthanol 90:10) de 0 à 100%. On obtient 0,4184g de *trans* (5-méthanesulfonylamino-adamantan-2-yl)-amide de l'acide 4-[5-(4-tert-butyl-pipérazin-1-yl)-pyridin-2-yl]-3,4-dihydro-2H-quinoxaline-1-carboxylique.
PF = 165°C.
M+H+ = 622-623.
1 H NMR (400 MHz, DMSO-d6) δ ppm = 8,02 (d, J = 3 Hz, 1 H), 7,44 (dd, J = 7,9 Hz et 1,5 Hz, 1H), 7,36 (dd, J = 9 Hz et 3 Hz, 1H), 7,10 (m, 2H), 6,93 (m, 1H), 6,86 (m, 2H), 6,06 (d, J = 5,6 Hz, 1H), 3,79 (m, 4H), 3,72 (m, 1H), 3,11 (m, 4H), 2,95 (s, 3H), 2,66 (m, 4H), 2,09 à 1,85 (m, 9H), 1,73 (m, 2H), 1,42 (m, 2H), 1,06 (m, 9H)

### Exemple 9 : Trans [5-(3-méthyl-[1,2,4]oxadiazol-5-yl)-adamantan-2-yl]-amide de l'acide 4-[5-(4-cyclopropanesulfonyl-pipérazin-1-yl)-pyridin-2-yl]-3,4-dihydro-2H-quinoxaline-1-carboxylique (composé n°10)

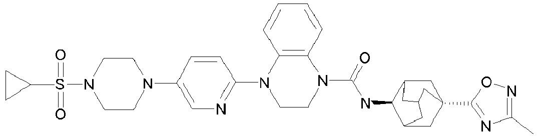

On place 0.149g de l'acide trans 4-({4-[5-(4-cyclopropanesulfonyl-pipérazin-1-yl)-pyridin-2-yl]-3,4-dihydro-2H-quinoxaline-1-carbonyl}-amino)-adamantane-1-carboxylique (intermédiaire 7.8) dans 2,4 ml de toluène, puis on ajoute 0,021 g de N-hydroxy-acétamidine, 0,044g de l'alcool benzotriazol-1-ol hydrate, 0,036 g de diisopropyl-carbodiimide et 2,4 ml de dichlorométhane, puis le mélange réactionnel est agité pendant 3h30 à température ambiante, puis est chauffé à 50°C pendant 18h. On rajoute 0,042g de N-hydroxy-acétamidine et on chauffe à 50°C pendant 18h. Le mélange réactionnel est concentré sous pression réduite, on y ajoute du dichlorométhane et de l'eau. La phase aqueuse est extraite trois fois par du dichlorométhane, la phase organique est lavée par de l'eau et une solution aqueuse saturée en chlorure de sodium. La phase organique est séchée sur sulfate de magnésium et est concentrée sous vide. On obtient 0,224g de trans (5-{1-[hydroxyimino]-éthylcarbamoyl}-adamantan-2-yl)-amide de l'acide 4-[5-(4-cyclopropanesulfonyl-pipérazin-1-yl)-pyridin-2-yl]-3,4-dihydro-2H-quinoxaline-1-carboxylique (M+H+ = 677) que l'on met dans 4,73ml d'un mélange (4:1) éthanol-eau avec 0,0271 g d'acétate de sodium. Le mélange réactionnel est chauffé à reflux 18h. On concentre sous pression réduite le mélange, on ajoute de l'eau et on extrait trois fois à l'acétate d'éthyle. La phase organique est séchée sur sulfate de magnésium et on concentre sous vide. Le brut obtenu est chromatographié sur gel de silice en éluant avec un mélange de solvants heptane/heptane-acétate d'éthyle-méthanol (4/5/1). On obtient 0,111 g de *trans* [5-(3-méthyl-[1,2,4]oxadiazol-5-yl)-adamantan-2-yl]-amide de l'acide 4-[5-(4-cyclopropanesulfonyl-pipérazin-1-yl)-pyridin-2-yl]-3,4-dihydro-2H-quinoxaline-1-carboxylique.
PF = 135°C ; M+H+ = 659.
1 H NMR (400 MHz, DMSO-d6) δ ppm = 8,08 (d, J = 3 Hz, 1 H), 7,49 (dd, J = 8 Hz et 1,5 Hz, 1H), 7,43 (dd, J = 9 Hz et 3 Hz, 1H), 7,15 (m, 2H), 6,95 (m, 1H), 6,89 (m, 1H), 6,19 (d, J = 5,7 Hz, 1H), 3,82 (m, 5H), 3,36 (m, 4H), 3,23 (m, 4H), 2,67 (m, 1H), 2,32 (s, 3H), 2,17 à 1,94 (m, 9H), 1,88 (m, 2H), 1,56 (m, 2H), 1,08 à 0,83 (m, 4H)

Le tableau qui suit illustre les structures chimiques et les propriétés physiques de quelques composés selon l'invention, répondant à la formule (I), dans laquelle R_{2b} représente un atome d'hydrogène, et se présentant sous forme de bases libres ou de composé salifié :
- dans la colonne « A », « - » représente une liaison simple ;
- base correspond à la molécule non salifiée
- dec. Correspond à une température de décomposition ;
- HCl représente un chlorhydrate
- Me représente un groupe méthyle ;
- PF représente le point de fusion du composé, exprimé en degrés Celsius ;
- Sel correspond à la forme du composé qui peut être sous forme de la base ou sous forme salifiée, par exemple un chlorhydrate ;
- M+H⁺ représente la masse du composé, obtenue par LC-MS (Liquid Chromatography Mass Spectroscopy).

**Tableau**

| | | | | | | | | | | | | | | | |
|---|---|---|---|---|---|---|---|---|---|---|---|---|---|---|---|
| 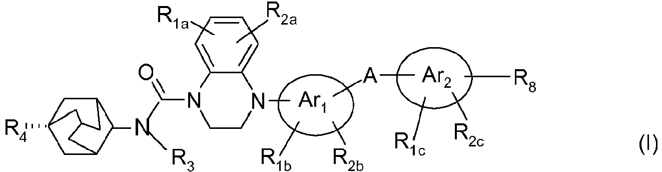 | | | | | | | | | | | | | | | |

| N° | A | R₁ₐ | R₂ₐ | R_{1b} | R_{1c} | R_{2c} | R₃ | R₄ | R₈ | Ar₁ | Ar₂ | Sel | PF (°C) | M+H⁺ | Synthèse |
|---|---|---|---|---|---|---|---|---|---|---|---|---|---|---|---|
| 1 | - | H | H | H | H | H | H | -CONH₂ *(cis)* | 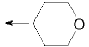 | 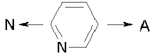 | 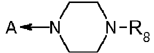 | Base | 144 | 600 | Méthode 1 |
| 2 | - | H | H | H | H | 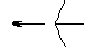 | H | -CH₂NHSO₂Me *(trans)* | H | 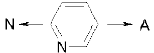 | 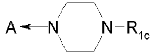 | HCl | 252 | 636 | Méthode 1 |
| 3 | - | H | H | H | H | 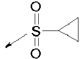 | H | -CH₂CONH₂ *(trans)* | H | 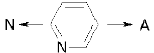 | 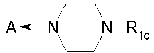 | Base | 120-135 | 634 | Méthode 1 |
| 4 | - | H | H | H | H | 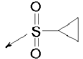 | H | -CH₂NH₂ *(trans)* | H | 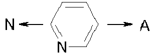 | 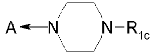 | HCl | 197 | 606 | Méthode 2 |
| 5 | - | H | H | H | H | 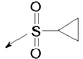 | H | -OCONH₂ *(trans)* | H | 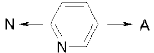 | 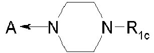 | Base | Dec. 106 | 636 | Méthode 4 |
| 6 | - | H | H | H | H | 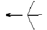 | H | -C(OH)(CF₃) *(trans)* | H | 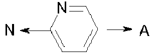 | 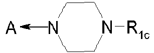 | Base | 192-196 | 627 | Méthode 3 |
| 7 | - | H | H | H | H | 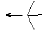 | H | 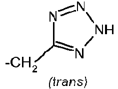 | H | 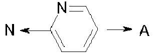 | 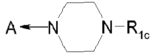 | Base | 160-170 | 611 | Méthode 1 |
| 8 | - | H | H | H | H | 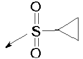 | H | -CONHOMe *(trans)* | H | 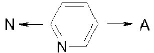 | 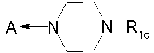 | Base | 125-163 | 650 | Méthode 5 |
| 9 | - | H | H | H | H | 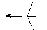 | H | -NHSO₂Me *(trans)* | H | 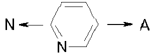 | 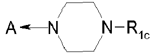 | Base | 165 | 62 | Méthode 1 |
| 10 | - | H | H | H | H | 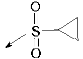 | H | 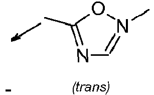 | H | 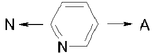 | 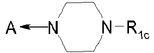 | Base | 135 | 659 | Méthode 6 |

Les composés selon l'invention ont fait l'objet d'essais pharmacologiques permettant de déterminer leur effet inhibiteur de l'enzyme 11βHSD1, qui intervient dans le métabolisme des lipides et le métabolisme du glucose.

Ces essais ont consisté à mesurer l'activité inhibitrice *in vitro* des composés de l'invention sur l'enzyme 11βHSD1 grâce à un test SPA (Scintillation Proximity Assay) en format 384 puits, La protéine 11βHSD1 recombinante a été produite en levure *S,cerevisiae,* La réaction a été réalisée en incubant l'enzyme en présence de ³H-cortisone et de NADPH, en absence ou en présence de concentration croissante d'inhibiteur, Des billes SPA couplées à un anticorps anti-souris, préincubées avec un anticorps anti-cortisol, ont permis de mesurer la quantité de cortisol formé au cours de la réaction.

L'activité inhibitrice vis-à-vis de l'enzyme 11βHSD1 est donnée par la concentration qui inhibe 50% de l'activité de 11βHSD1 (Cl₅₀).

Les Cl₅₀ des composés de l'invention sont reportés dans le tableau ci-après :

| Composé n° | 11βHSD1-HR Cl₅₀ nM |
|---|---|
| 1 | 68 |
| 2 | 32 |
| 3 | 21 |
| 4 | 23 |
| 5 | 11 |
| 6 | 57 |
| 7 | 45 |
| 8 | 204 |
| 9 | 31 |
| 10 | 720 |

Il apparaît donc que les composés selon l'invention ont une activité inhibitrice de l'enzyme 11βHSD1. Les composés selon l'invention peuvent donc être utilisés pour la préparation de médicaments, en particulier de médicaments inhibiteurs de l'enzyme 11βHSD1.

Ainsi, selon un autre de ses aspects, l'invention a pour objet des médicaments qui comprennent un composé de formule (I), ou un sel d'addition de ce dernier à un acide ou à une base pharmaceutiquement acceptable, ou encore un hydrate ou un solvat du composé de formule (I).

Ces médicaments trouvent leur emploi en thérapeutique, notamment dans le traitement et la prévention de l'obésité, des diabètes, des troubles de la microcirculation, de la résistance à l'insuline, du syndrome métabolique, du syndrome de Cushing, de l'hypertension, de l'athérosclérose, de la cognition et de la démence, des glaucomes, de l'ostéoporose, de la lipodystrophie, de l'hypertrophie cardiaque, de l'insuffisance cardiaque, de maladies hépatiques, de certaines maladies infectieuses en augmentant l'efficacité du système immunitaire ou encore pour favoriser la cicatrisation de plaies.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale, telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants, Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 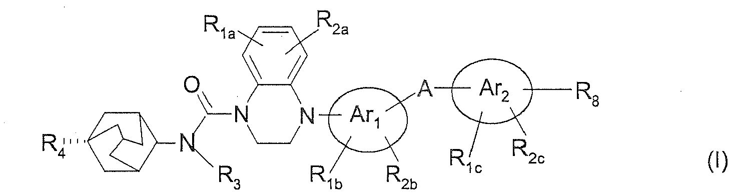 dans laquelle :
- A représente une liaison, un atome d'oxygène ou un groupe -O-CH₂-,
- Ar₁ représente un groupe phényle ou hétéroaryle,
- Ar₂ représente un groupe phényle, un groupe hétéroaryle ou un groupe hétérocycloalkyle,
- R_{1a,b,c} et R_{2a,b,c}, identiques ou différents, représentent chacun un atome d'hydrogène ou d'halogène ou un groupe alkyle, cycloalkyle, -alkyl-cycloalkyle éventuellement substitué par un ou plusieurs atomes d'halogène, -OR₅ (hydroxy ou alcoxy), hydroxy-alkyle, alcoxy-alkyle, alcoxy-alcoxy, halogénoalkyle, -O-halogénoalkyle, oxo, -CO-alkyle, -CO-alkyle-NR₆R₇, -CO-halogénoalkyle, -COOR₅, alkyl-COOR₅, -O-alkyl-COOR₅, -SO₂-alkyle, -SO₂-cycloalkyle, -SO₂-alkyl-cycloalkyle, -SO₂-alkyle-OR₅, -SO₂-alkyl-COOR₅, -SO₂-alkyl-NR₆R₇, -SO₂-halogénoalkyle, alkyl-SO₂-alkyle, -SO₂-NR₆R₇, -SO₂-alkyl-alcoxy-alcoxy, -CONR₆R₇, -alkyl-CONR₆R₇ ou -O-alkyl-NR₆R₇, ou encore R₁ₐ, R_{1b}, R_{1c} sont liés respectivement à R₂ₐ, R_{2b,} R_{2c} et à l'atome de carbone qui les porte et représentent -O-alkyl-O- ;
- R₃ représente un atome d'hydrogène ou un groupe alkyle,
- R₄ représente un groupe -CONR₆R₇; hydroxy-alkyle substitué par un groupe halogénoalkyle ; -alkyl-NH-SO₂-alkyle ; -NH-SO₂-alkyle ; -O-SO₂-NR₆R₇ ; -alkyl-CO-NR₆R₇ ; -O-alkyl-CO-NR₆R₇ ; -alkyl-NR₆R₇ ; -O-CO-NR₆R₇ ; -alkyl-hétéroaryle ; hétéroaryle éventuellement substitué par un groupe alkyle ; alcoxy-imino ; -CO-NH-NH-CO-alkyle; à la condition que R₄ soit en position cis lorsqu'il représente le groupe -CONR₆R₇ et que R₆ et R₇ représentent chacun l'hydrogène, un groupe -alkyle ou -alkyle-phényle ;
- R₅, représentent l'hydrogène, un groupe alkyle ou un groupe -alkyl-phényle,
- R₆ et R₇, identiques ou différents, représentent chacun un atome d'hydrogène, un groupe alkyle, alcoxy ou un groupe -alkyl-phényle, et
- R₈ représente un atome d'hydrogène, un groupe alkyle ou un groupe de formule -B-Het, où B peut être absent ou représenter une liaison, un atome d'oxygène ou un groupe -CO- ou -SO₂-(CH₂)ₙ avec n égal à 0, 1 ou 2 et où Het représente un hétéroaryle ou un hétérocycloalkyle éventuellement substitué par 1 à 3 groupes choisis parmi les groupes alkyles, -SO₂-alkyles et -COOR₅,
à l'état de base ou d'acide ou de sel d'addition à un acide ou à une base.

2. Composé de formule (I) selon la revendication 1, **caractérisé en ce que** A représente une liaison.

3. Composé de formule (I) selon l'une des revendications 1 ou 2, **caractérisé en ce que** Ar₁ représente un groupe hétéroaryle

4. Composé de formule (I) selon la revendication 3, **caractérisé en ce que** Ar₁ représente le groupe pyridinyle.

5. Composé de formule (I) selon l'une quelconque des revendications 1 à 4, **caractérisé en ce que** Ar₂ représente un groupe hétérocycloalkyle.

6. Composé de formule (I) selon la revendication 4, **caractérisé en ce que** Ar₂ représente le groupe pipérazinyle.

7. Composé de formule (I) selon l'une quelconque des revendications 1 à 6, **caractérisé en ce que** R₁ₐ, R₂ₐ, R_{1b} et R_{2b}, représentent chacun un atome d'hydrogène.

8. Composé de formule (I) selon l'une quelconque des revendications 1 à 7, **caractérisé en ce que** R_{1c} et R_{2c}, identiques ou différents, représentent chacun un atome d'hydrogène, un groupe alkyle ou -SO₂-cycloalkyle.

9. Composé de formule (I) selon l'une quelconque des revendications 1 à 8, **caractérisé en ce que** R_{1a,b} et R_{2a,b,c}, représentent chacun un atome d'hydrogène et R_{1c} représente l'hydrogène, un groupe alkyle, ou -SO₂-cycloalkyle.

10. Composé de formule (I) selon l'une quelconque des revendications 1 à 9, **caractérisé en ce que** R₃ représente un atome d'hydrogène.

11. Composé de formule (I) selon l'une quelconque des revendications 1 à 10, **caractérisé en ce que** R₈ représente un atome d'hydrogène ou un groupe de formule -B-Het, où B peut être absent et Het représente un groupe tétrahydropyranyle.

12. Composé de formule (I) selon l'une quelconque des revendications 1 à 11, **caractérisé en ce que** :
- A est une liaison ;
- Ar₁ est un hétéroaryle ;
- Ar₂ est un hétérocycloalkyle ;
- R₃ représente un atome d'hydrogène ou un alkyle,
- R₄ représente un groupe -CONR₆R₇ ; hydroxy-alkyle substitué par un groupe halogénoalkyle ; -alkyl-NH-SO₂-alkyle ; -NH-SO₂-alkyle ; -O-SO₂-NR₆R₇; -alkyl-CO-NR₆R₇; -O-alkyl-CO-NR₆R₇; -alkyl-NR₆R₇; -O-CO-NR₆R₇; -alkyl-hétéroaryle ; hétéroaryle éventuellement substitué par un groupe alkyle ; alcoxy-imino ; -CO-NH-NH-CO-alkyle; à la condition que R₄ soit en position cis lorsqu'il représente le groupe -CONR₆R₇ et que R₆ et R₇ représentent chacun l'hydrogène, un groupe -alkyle ou -alkyle-phényle ;
- R₅, représentent l'hydrogène, un groupe alkyle ou un groupe -alkyl-phényle,
- R₆ et R₇, identiques ou différents, représentent chacun un atome d'hydrogène, un groupe alkyle, alcoxy ou un groupe -alkyl-phényle, et
- R₈ représente un atome d'hydrogène ; un groupe alkyle ou un groupe tétrahydropyranyle.

13. Composé de formule (I) selon l'une quelconque des revendications 1 à 12, **caractérisé en ce que** R_{1a,b},ₑ et R_{2a,b,c} sont l'hydrogène et R₈ est un groupe alkyle ou tétrahydropyranyle.

14. Composé de formule (I) selon l'une quelconque des revendications 1 à 13, **caractérisé en ce que** R_{1a,b,c} et R_{2a,b} et R₈ sont l'hydrogène et R_{2c} est un groupe -SO₂- cycloalkyle.

15. Composé de formule (I) selon la revendication 1, **caractérisé en ce qu'**il est choisi parmi :
Acide Cis 4-{5-[4-(Tetrahydro-pyran-4-yl)-piperazin-1-yl]-pyridin-2-yl}-3,4-dihydro-2H-quinoxaline-1-carboxylique(5-carbamoyl-adamantan-2-yl)-amide ;
Acide Trans 4-[5-(4-tert-Butyl-piperazin-1-yl)-pyridin-2-yl]-3,4-dihydro-2H-quinoxaline-1-carboxylique[5-(methanesulfonylamino-methyl)-adamantan-2-yl]-amide ;
Acide Trans 4-[5-(4-Cyclopropanesulfonyl-piperazin-1-yl)-pyridin-2-yl]-3,4-dihydro-2H-quinoxaline-1-carboxylique(5-carbamoylmethyl-adamantan-2-yl)-amide ;
Acide Trans 4-[5-(4-Cyclopropanesulfonyl-piperazin-1-yl)-pyridin-2-yl]-3,4-dihydro-2H-quinoxaline-1-carboxylique(5-aminomethyl-adamantan-2-yl)-amide ;
Acide Trans Carbamique 4-({4-[5-(4-cyclopropanesulfonyl-piperazin-1-yl)-pyridin-2-yl]-3,4-dihydro-2H-quinoxaline-1-carbonyl}-amino)-adamantan-1-yl ester ;
Acide Trans 4-[5-(4-tert-Butyl-piperazin-1-yl)-pyridin-2-yl]-3,4-dihydro-2H-quinoxaline-1-carboxylique[5-(2,2,2-trifluoro-1-hydroxy-ethyl)-adamantan-2-yl]-amide ;
Acide Trans 4-[5-(4-tert-Butyl-piperazin-1-yl)-pyridin-2-yl]-3,4-dihydro-2H-quinoxaline-1-carboxylique[5-(2H-tetrazol-5-ylmethyl)-adamantan-2-yl]-amide ;
Acide Trans 4-[5-(4-Cyclopropanesulfonyl-piperazin-1-yl)-pyridin-2-yl]-3,4-dihydro-2H-quinoxaline-1-carboxylique(5-methoxycarbamoyl-adamantan-2-yl)-amide ;
Acide Trans 4-[5-(4-tert-Butyl-piperazin-1-yl)-pyridin-2-yl]-3,4-dihydro-2H-quinoxaline-1-carboxylique(5-methanesulfonylamino-adamantan-2-yl)-amide.
Acide Trans 4-[5-(4-Cyclopropanesulfonyl-piperazin-1-yl)-pyridin-2-yl]-3,4-dihydro-2H-quinoxaline-1-carboxylique[5-(3-methyl-[1,2,4]oxadiazol-5-yl)-adamantan-2-yl]-amide

16. Médicament, **caractérisé en ce qu'**il comprend un composé de formule (I) selon l'une quelconque des revendications 1 à 15, ou un sel d'addition de ce composé à un acide ou à une base pharmaceutiquement acceptable, ou encore un solvat du composé de formule (I).

17. Composition pharmaceutique, **caractérisée en ce qu'**elle comprend un composé de formule (I) selon l'une quelconque des revendications 1 à 15, ou un sel pharmaceutiquement acceptable ou un solvat de ce composé, ainsi qu'au moins un excipient pharmaceutiquement acceptable.

18. Composé de formule (I) selon l'une quelconque des revendications 1 à 15 pour son utilisation dans le traitement et la prévention de l'obésité, des diabètes, des troubles de la microcirculation, de la résistance à l'insuline, du syndrome métabolique, du syndrome de Cushing, de l'hypertension, de l'athérosclérose, de la cognition et de la démence, des glaucomes, de l'ostéoporose, de la lipodystrophie, de l'hypertrophie cardiaque, de l'insuffisance cardiaque, de maladies hépatiques, de certaines maladies infectieuses en augmentant l'efficacité du système immunitaire ou des plaies en favorisant la cicatrisation.

## Patentansprüche

1. Verbindung, entsprechend der Formel (I): 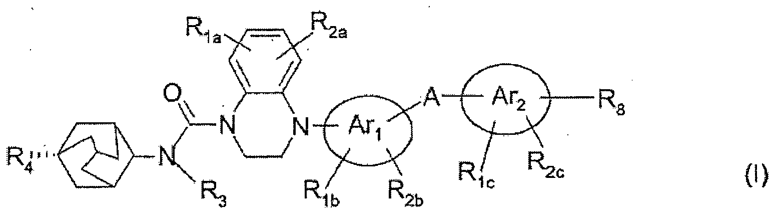 in der:
- A eine Bindung, ein Sauerstoffatom oder eine -O-CH₂-Gruppe darstellt,
- Ar₁ eine Phenyl- oder Heteroarylgruppe darstellt,
- Ar₂ eine Phenylgruppe, eine Heteroarylgruppe oder eine Heterocycloalkylgruppe darstellt,
- R_{1a,b,c} und R_{2a,b,c}, die gleich oder verschieden sind, jeweils ein Wasserstoff- oder Halogenatom oder eine Alkyl-, Cycloalkyl-, Alkyl-Cycloalkylgruppe, gegebenenfalls mit einem oder mehreren Halogenatomen substituiert, -OR₅ (Hydroxy oder Alkoxy), Hydroxy-Alkyl, Alkoxy-Alkyl, Alkoxy-Alkoxy, Halogenalkyl, -O-Halogenalkyl, Oxo, -CO-Alkyl, -CO-Alkyl-NR₆R₇, -CO-Halogenalkyl, -COOR₅, -Alkyl-COOR₅, -O-Alkyl-COOR₅, -SO₂-Alkyl, -SO₂-Cycloalkyl, -SO₂-Alkyl-Cycloalkyl, -SO₂-Alkyl-OR₅, -SO₂-Alkyl-COOR₅, -SO₂-Alkyl-NR₆R₇, -SO₂-Halogenalkyl, Alkyl-SO₂-Alkyl, -SO₂-NR₆R₇, -SO₂-Alkyl-Alkoxy-Alkoxy, -CONR₆R₇, -Alkyl-CONR₆R₇ oder -O-Alkyl-NR₆R₇ darstellen, oder R₁ₐ, R_{1b}, R_{1c} jeweils an R₂ₐ, R_{2b}, R_{2c} und an das Kohlenstoffatom, welches sie trägt, gebunden sind und -O-Alkyl-O- darstellen;
- R₃ ein Wasserstoffatom oder eine Alkylgruppe darstellt,
- R₄ eine -CONR₆R₇-Gruppe; Hydroxy-Alkyl, substituiert mit einer Halogenalkylgruppe; -Alkyl-NH-SO₂-Alkyl; -NH-SO₂-Alkyl; -O-SO₂-NR₆R₇; -Alkyl-CO-NR₆R₇; -O-Alkyl-CO-NR₆R₇; -Alkyl-NR₆R₇; -O-CO-NR₆R₇; -Alkyl-Heteroaryl; Heteroaryl, gegebenenfalls substituiert mit einer Alkylgruppe; Alkoxy-imino; -CO-NH-NH-CO-Alkyl darstellt; unter der Bedingung, dass R₄ in cis-Stellung ist, wenn es die Gruppe -CON-R₆R₇ darstellt, und dass R₆ und R₇ jeweils Wasserstoff, eine Gruppe -Alkyl oder -Alkyl-Phenyl darstellen;
- R₅ Wasserstoff, eine Allylgruppe oder eine Gruppe -Alkyl-Phenyl darstellt,
- R₆ und R₇, die gleich oder verschieden sind, jeweils ein Wasserstoffatom, eine Alkyl-, Alkoxygruppe oder eine Gruppe -Alkyl-Phenyl darstellen, und
- R₈ ein Wasserstoffatom, eine Alkylgruppe oder eine Gruppe der Formel -B-Het darstellt, wobei B abwesend sein oder eine Bindung, ein Sauerstoffatom oder eine Gruppe -CO- oder -SO₂-(CH₂)ₙ- mit n gleich 0, 1 oder 2 darstellen kann, und wobei Het ein Heteroaryl oder ein Heterocycloalkyl, gegebenenfalls substituiert mit 1 bis 3 Gruppen, ausgewählt aus den Gruppen Alkyl, SO₂-Alkyl und -COOR₅, darstellt, in Form einer Base oder einer Säure oder eines Additionssalzes einer Säure oder einer Base.

2. Verbindung der Formel (I) nach Anspruch 1, **dadurch gekennzeichnet, dass** A eine Bindung darstellt.

3. Verbindung der Formel (I) nach einem der Ansprüche 1 oder 2, **dadurch gekennzeichnet, dass** Ar₁ eine Heteroarylgruppe darstellt.

4. Verbindung der Formel (I) nach Anspruch 3, **dadurch gekennzeichnet, dass** Ar₁ die Pyridinylgruppe darstellt.

5. Verbindung der Formel (I) nach einem der Ansprüche 1 bis 4, **dadurch gekennzeichnet, dass** Ar₂ eine Heterocycloalkylgruppe darstellt.

6. Verbindung der Formel (I) nach Anspruch 4, **dadurch gekennzeichnet, dass** Ar₂ die Piperazinylgruppe darstellt.

7. Verbindung der Formel (I) nach einem der Ansprüche 1 bis 6, **dadurch gekennzeichnet, dass** R₁ₐ, R₂ₐ, R_{1b} und R_{2b}, jeweils ein Wasserstoffatom darstellen.

8. Verbindung der Formel (I) nach einem der Ansprüche 1 bis 7, **dadurch gekennzeichnet, dass** R_{1c} und R_{2c}, die gleich oder verschieden sind, jeweils ein Wasserstoffatom, eine Alkylgruppe oder -SO₂-Cycloalkyl darstellen.

9. Verbindung der Formel (I) nach einem der Ansprüche 1 bis 8, **dadurch gekennzeichnet, dass** R_{1a,b} und R_{2a,b,c} jeweils ein Wasserstoffatom darstellen und R_{1c} Wasserstoff, eine Alkylgruppe oder -SO₂-Cycloalkyl darstellt.

10. Verbindung der Formel (I) nach einem der Ansprüche 1 bis 9, **dadurch gekennzeichnet, dass** R₃ ein Wasserstoffatom darstellt.

11. Verbindung der Formel (I) nach einem der Ansprüche 1 bis 10, **dadurch gekennzeichnet, dass** R₈ ein Wasserstoffatom oder eine Gruppe der Formel -B-Het darstellt, wobei B abwesend sein kann und Het eine Tetrahydropyranylgruppe darstellt.

12. Verbindung der Formel (I) nach einem der Ansprüche 1 bis 11, **dadurch gekennzeichnet, dass**:
- A eine Bindung ist;
- Ar₁ ein Heteroaryl ist;
- Ar₂ ein Heterocycloalkyl ist;
- R₃ ein Wasserstoffatom oder ein Alkyl darstellt,
- R₄ eine -CONR₆R₇-Gruppe; Hydroxy-Alkyl, substituiert mit einer Halogenalkylgruppe; -Alkyl-NH-SO₂-Alkyl; -NH-SO₂-Alkyl; -O-SO₂-NR₆R₇; -Alkyl-CO-NR₆R₇; -O-Alkyl-CO-NR₆R₇; -Alkyl-NR₆R₇; -O-CO-NR₆R₇; -Alkyl-Heteroaryl; Heteroaryl, gegebenenfalls substituiert mit einer Alkylgruppe; Alkoxy-imino; -CO-NH-NH-CO-Alkyl darstellt; unter der Bedingung, dass R₄ in cis-Stellung ist, wenn es die Gruppe -CONR₆R₇ darstellt, und dass R₆ und R₇ jeweils Wasserstoff, eine Gruppe -Alkyl oder -Alkyl-Phenyl darstellen;
- R₅ Wasserstoff, eine Alkylgruppe oder eine Gruppe -Alkyl-Phenyl darstellt,
- R₆ und R₇, die gleich oder verschieden sind, jeweils ein Wasserstoffatom, eine Alkyl-, Alkoxygruppe oder eine Gruppe -Alkyl-Phenyl darstellen, und
- R₈ ein Wasserstoffatom, eine Alkylgruppe oder eine Tetrahydropyranylgruppe darstellt.

13. Verbindung der Formel (I) nach einem der Ansprüche 1 bis 12, **dadurch gekennzeichnet, dass** R_{1a,b,c} und R_{2a,b,c} und R₈ Wasserstoff sind und R₈ eine Alkyl- oder Tetrahydropyranylgruppe ist.

14. Verbindung der Formel (I) nach einem der Ansprüche 1 bis 13, **dadurch gekennzeichnet, dass** R_{1a,b,c} und R_{2a,b} Wasserstoff sind und R_{2c} eine -SO₂-Cycloalkylgruppe ist.

15. Verbindung der Formel (I) nach Anspruch 1, **dadurch gekennzeichnet, dass** sie ausgewählt ist aus:
Cis-4-{5-[4-(Tetrahydro-pyran-4-yl)-piperazin-1-yl]-pyridin-2-yl}-3,4-dihydro-2H-chinoxalin-1-carbonsäure-(5-carbamoyl-adamantan-2-yl)-amid;
Trans-4-[5-(4-tert-Butyl-piperazin-1-yl)-pyridin-2-yl]-3,4-dihydro-2H-chin-oxalin-1-carbonsäure-[5-(methansulfonylamino-methyl)-adamantan-2-yl]-amid;
Trans-4-[5-(4-Cyclopropansulfonyl-piperazin-1-yl)-pyridin-2-yl]-3,4-dihydro-2H-chinoxalin-1-carbonsäure-(5-carbamoylmethyl-adamantan-2-yl)-amid;
Trans-4-[5-(4-Cyclopropansulfonyl-piperazin-1-yl)-pyridin-2-yl]-3,4-dihydro-2H-chinoxalin-1-carbonsäure-(5-aminomethyl-adamantan-2-yl)-amid;
Trans-carbamidsäure-4-({4-[5-(4-cyclopropansulfonyl-piperazin-1-yl)-pyridin-2-yl]-3,4-dihydro-2H-chinoxalin-1-carbonyl}-amino)-adamantan-1-yl-ester;
Trans-4-[5-(4-tert-Butyl-piperazin-1-yl)-pyridin-2-yl]-3,4-dihydro-2H-chin-oxalin-1-carbonsäure-[5-(2,2,2-trifluor-1-hydroxy-ethyl)-adamantan-2-yl]-amid;
Trans-4-[5-(4-tert-Butyl-piperazin-1-yl)-pyridin-2-yl]-3,4-dihydro-2H-chin-oxalin-1-carbonsäure-[5-(2H-tetrazol-5-ylmethyl)-adamantan-2-yl] amid;
Trans-4-[5-(4-Cyclopropansulfonyl-piperazin-1-yl)-pyridin-2-yl]-3,4-dihy-dro-2H-chinoxalin-1-carbonsäure-(5-methoxycarbamoyl-adamantan-2-yl)-amid;
Trans-4-[5-(4-tert-Butyl-piperazin-1-yl)-pyridin-2-yl]-3,4-dihydro-2H-chin-oxalin-1-carbonsäure-(5-methansulfonylamino-adamantan-2-yl)-amid;
Trans-4-[5-(4-Cyclopropansulfonyl-piperazin-1-yl)-pyridin-2-yl]-3,4-dihy-dro-2H-chinoxalin-1-carbonsäure-[5-(3-methyl-[1,2,4]oxadiazol-5-yl)-adamantan-2-yl]-amid.

16. Arzneimittel, **dadurch gekennzeichnet, dass** es eine Verbindung der Formel (I) nach einem der Ansprüche 1 bis 15 oder ein pharmazeutisch annehmbares Additionssalz dieser Verbindung einer Säure oder einer Base oder ein Solvat der Verbindung der Formel (I) umfasst.

17. Pharmazeutische Zusammensetzung, **dadurch gekennzeichnet, dass** sie eine Verbindung der Formel (I) nach einem der Ansprüche 1 bis 15 oder ein pharmazeutisch annehmbares Salz oder ein Solvat dieser Verbindung, sowie mindestens einen pharmazeutisch annehmbaren Hilfsstoff umfasst.

18. Verbindung der Formel (I) nach nach einem der Ansprüche 1 bis 15 für seine Verwendung in der Behandlung und Vorbeugung von Obesitas, Diabetes, Störungen der Mikrozirkulation, Insulinresistenz, metabolischem Syndrom, Cushing-Syndrom, Bluthochdruck, Atherosklerose, Kognition und Demenz, Glaukomen, Osteoporose, Lipodystrophie, kardiale Hypertrophie, Herzinsuffizienz, Lebererkrankungen, bestimmten infektiösen Erkrankungen durch die Steigerung der Effizienz des Immunsystems oder von Wunden durch Förderung der Wundheilung.

## Claims

1. Compound corresponding to formula (I): 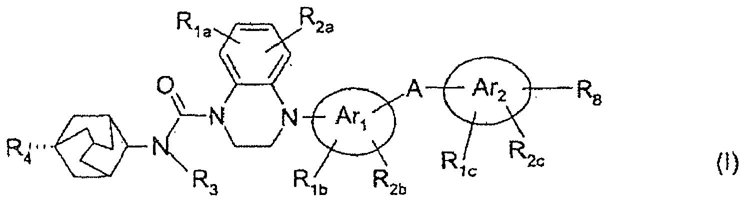 in which:
- A represents a bond, an oxygen atom or an -O-CH₂-group,
- Ar₁ represents a phenyl or heteroaryl group,
- Ar₂ represents a phenyl group, a heteroaryl group or a heterocycloalkyl group,
- R_{1a,b,c} and R_{2a,b,c}, which may be identical or different, each represent a hydrogen or halogen atom or an alkyl group, cycloalkyl, -alkyl-cycloalkyl optionally substituted with one or more halogen atoms; -OR₅ (hydroxy or alkoxy); hydroxy-alkyl; alkoxy-alkyl; alkoxy-alkoxy; haloalkyl; -O-haloalkyl; oxo; -CO-alkyl; -CO-alkyl-NR₆R₇; -CO-haloalkyl; -COOR₅; alkyl-COOR₅; -O-alkyl-COOR₅; -SO₂-alkyl; -SO₂-cycloalkyl; -SO₂-alkyl-cycloalkyl; -SO₂-alkyl-OR₅; -SO₂-alkyl-COOR₅; -SO₂-alkyl-NR₆R₇; -SO₂-haloalkyl; alkyl-SO₂-alkyl; -SO₂-NR₆R₇; -SO₂-alkyl-alkoxy-alkoxy; -CONR₆R₇; -alkyl-CONR₆R₇ or -O-alkyl-NR₆R₇, or R₁ₐ, R_{1b}, R_{1c} are bound respectively to R₂ₐ, R_{2b}, R_{2c} and to the carbon atom that bears them and represent -O-alkyl-O-;
- R₃ represents a hydrogen atom or an alkyl group,
- R₄ represents a group -CONR₆R₇; hydroxy-alkyl substituted with a haloalkyl group; -alkyl-NH-SO₂-alkyl ; -NH-SO₂-alkyl; -O-SO₂-NR₆R₇; -alkyl-CO-NR₆R₇; -O-alkyl-CO-NR₆R₇; -alkyl-NR₆R₇; -O-CO-NR₆R₇; alkyl-heteroaryl; heteroaryl optionally substituted with an alkyl group; alkoxy-imino; -CO-NH-NH-CO-alkyl; provided that R₄ is in cis position when it represents the group -CONR₆R₇ and that R₆ and R₇ each represent hydrogen, an -alkyl or - alkyl-phenyl group;
- R₅ represent hydrogen, an alkyl group or an - alkyl-phenyl group;
- R₆ and R₇, which may be identical or different, each represent a hydrogen atom, an alkyl or alkoxy group or an -alkyl-phenyl group, and
- R₈ represents a hydrogen atom, an alkyl group or a group of formula -B-Het, where B can be absent or can represent a bond, an oxygen atom or a -CO- or -SO₂-(CH₂)ₙ group with in equal to 0, 1 or 2 and where Het represents a heteroaryl or a heterocycloalkyl optionally substituted with 1 to 3 groups selected from the alkyl, -SO₂-alkyl and -COOR₅ groups,
in the form of base or of acid or of salt of addition with an acid or with a base.

2. Compound of formula (I) according to Claim 1, **characterized in that** A represents a bond.

3. Compound of formula (I) according to either one of Claims 1 and 2, **characterized in that** Ar₁ represents a heteroaryl group.

4. Compound of formula (I) according to Claim 3, **characterized in that** Ar₁ represents the pyridinyl group.

5. Compound of formula (I) according to any one of Claims 1 to 4, **characterized in that** Ar₂ represents a heterocycloalkyl group.

6. Compound of formula (I) according to Claim 4, **characterized in that** Ar₂ represents the piperazinyl group.

7. Compound of formula (I) according to any one of Claims 1 to 6, **characterized in that** R₁ₐ, R₂ₐ, R_{1b} and R_{2b}, each represent a hydrogen atom.

8. Compound of formula (I) according to any one of Claims 1 to 7, **characterized in that** R_{1c} and R_{2c}, which may be identical or different, each represent a hydrogen atom, an alkyl or -SO₂-cycloalkyl group.

9. Compound of formula (I) according to any one of Claims 1 to 8, **characterized in that** R_{1a,b} and R_{2a,b,c} each represent a hydrogen atom and R_{1c} represents hydrogen, an alkyl, or -SO₂-cycloalkyl group.

10. Compound of formula (I) according to any one of Claims 1 to 9, **characterized in that** R₃ represents a hydrogen atom.

11. Compound of formula (I) according to any one of Claims 1 to 10, **characterized in that** R₈ represents a hydrogen atom or a group of formula -B-Het, where B can be absent and Het represents a tetrahydropyranyl group.

12. Compound of formula (I) according to any one of Claims 1 to 11, **characterized in that**:
- A is a bond;
- Ar₁ is a heteroaryl;
- Ar₂ is a heterocycloalkyl;
- R₃ represents a hydrogen atom or an alkyl,
- R₄ represents a group -CONR₆R₇; hydroxy-alkyl substituted with a haloalkyl group; -alkyl-NH-SO₂-alkyl; -NH-SO₂-alkyl; -O-SO₂-NR₆R₇; -alkyl-CO-NR₆R₇; -O-alkyl-CO-NR₆R₇; -alkyl-NR₆R₇; -O-CO-NR₆R₇; alkyl-heteroaryl; heteroaryl optionally substituted with an alkyl group; alkoxy-imino; -CO-NH-NH-CO-alkyl; provided that R₄ is in cis position when it represents the group -CONR₆R₇ and that R₆ and R₇ each represent hydrogen, an -alkyl or -alkyl-phenyl group;
- R₅ represent hydrogen, an alkyl group or an -alkyl-phenyl group;
- R₆ and R₇, which may be identical or different, each represent a hydrogen atom, an alkyl or alkoxy group or an -alkyl-phenyl group, and
- R₈ represents a hydrogen atom; an alkyl group or a tetrahydropyranyl group.

13. Compound of formula (I) according to any one of Claims 1 to 12, **characterized in that** R_{1a,b,c} and R_{2a,b,c} are hydrogen and R₈ is an alkyl or tetrahydropyranyl group.

14. Compound of formula (I) according to any one of Claims 1 to 13, **characterized in that** R_{1a,b,c} and R_{2a,b} and R₈ are hydrogen and R_{2c} is an -SO₂-cycloalkyl group.

15. Compound of formula (I) according to Claim 1, **characterized in that** it is selected from:
Cis 4-{5-[4-(Tetrahydro-pyran-4-yl)piperazin-1-yl]pyridin-2-yl}-3,4-dihydro-2H-quinoxaline-1-carboxylic acid (5-carbamoyl-adamantan-2-yl)amide;
Trans 4-[5-(4-tert-Butyl-piperazin-1-yl)pyridin-2-yl]-3,4-dihydro-2H-quinoxaline-1-carboxylic acid [5-(methanesulfonylamino-methyl)adamantan-2-yl]amide;
Trans 4-[5-(4-Cyclopropanesulfonyl-piperazin-1-yl)pyridin-2-yl]-3,4-dihydro-2H-quinoxaline-1-carboxylic acid (5-carbamoylmethyl-adamantan-2-yl)amide;
Trans 4-[5-(4-Cyclopropanesulfonyl-piperazin-1-yl)pyridin-2-yl]-3,4-dihydro-2H-quinoxaline-1-carboxylic acid (5-aminomethyl-adamantan-2-yl)amide;
Trans Carbamic acid 4-({4-[5-(4-cyclopropanesulfonyl-piperazin-1-yl)pyridin-2-yl]-3,4-dihydro-2H-quinoxaline-1-carbonyl}amino)adamantan-1-yl ester;
Trans 4-[5-(4-tert-Butyl-piperazin-1-yl)pyridin-2-yl]-3,4-dihydro-2H-quinoxaline-1-carboxylic acid [5-(2,2,2-trifluoro-1-hydroxy-ethyl)adamantan-2-yl]amide;
Trans 4-[5-(4-tert-Butyl-piperazin-1-yl)pyridin-2-yl]-3,4-dihydro-2H-quinoxaline-1-carboxylic acid [5-(2H-tetrazol-5-ylmethyl)adamantan-2-yl]amide;
Trans 4-[5-(4-Cyclopropanesulfonyl-piperazin-1-yl)pyridin-2-yl]-3,4-dihydro-2H-quinoxaline-1-carboxylic acid (5-methoxycarbamoyl-adamantan-2-yl)amide;
Trans 4-[5-(4-tert-Butyl-piperazin-1-yl)pyridin-2-yl]-3,4-dihydro-2H-quinoxaline-1-carboxylic acid (5-methanesulfonylamino-adamantan-2-yl)amide;
Trans 4-[5-(4-Cyclopropanesulfonyl-piperazin-1-yl)pyridin-2-yl]-3,4-dihydro-2H-quinoxaline-1-carboxylic acid [5-(3-methyl-[1,2,4]oxadiazol-5-yl)adamantan-2-yl]amide.

16. Medicinal product, **characterized in that** it comprises a compound of formula (I) according to any one of Claims 1 to 15, or a salt of addition of this compound with a pharmaceutically acceptable acid or base, or a solvate of the compound of formula (I).

17. Pharmaceutical composition, **characterized in that** it comprises a compound of formula (I) according to any one of Claims 1 to 15, or a pharmaceutically acceptable salt or a solvate of said compound, as well as at least one pharmaceutically acceptable excipient.

18. Compound of formula (I) according to any one of Claims 1 to 15 for use in the treatment and prevention of obesity, diabetes, microcirculatory disorders, insulin resistance, metabolic syndrome, Cushing syndrome, hypertension, atherosclerosis, cognition and dementia, glaucomas, osteoporosis, lipodystrophy, cardiac hypertrophy, heart failure, liver diseases, and of certain infectious diseases by increasing the effectiveness of the immune system or of wounds by promoting healing.
